# EUROPEAN PATENT APPLICATION

(11) **EP 4 293 057 A1**
(43) Date of publication of application: **20.12.2023**
(21) Application number: 22178893.8
(22) Date of filing: 14.06.2022
(51) Int. Cl.: C08F 220/28, C08F 220/58, A61K 9/00, A61K 31/78, A61P 31/14, A61P 31/20, C08F 293/00, C08F 8/36

(54) **POLYMER FOR INHIBITING SARS-COV-2 AND OTHER PATHOGENS**

(71) Applicant: Freie Universität Berlin, 14195 Berlin (DE)
(72) Inventor: HAAG, Rainer, 12209 Berlin (DE); BEJ, Raju, 14129 Berlin (DE)
(74) Representative: Maikowski & Ninnemann Patentanwälte Partnerschaft mbB

(57) **Abstract**

The present invention relates to methacrylate-based functionalized dendronized polyglycerol polymers. These polymers are highly biocompatible and less anticoagulant, form thread-like single chain fibers and show excellent inhibition against respiratory viruses such as SARS-CoV-2 and HSV-1. They can be easily used for forming degradable hydrogels together with a crosslinker.

## Description

The present invention relates to a polymer according to the preamble of claim 1, to a hydrogel according to the preamble of claim 6 that can be obtained from such a polymer, to various uses of such a polymer and such a hydrogel according to the preambles of claims 7 to 11, to a pharmaceutical composition comprising such a polymer or such a hydrogel according to the preamble of claim 12, and to various manufacturing methods for such a polymer according to the preambles of claims 13 to 15.

The ongoing coronavirus disease (COVID-19) pandemic caused by severe acute respiratory syndrome coronavirus 2 (SARS-CoV-2) motivates scientists to develop compounds that help against a COVID-19 infection or limit the negative consequences of such infection. In this regard, the development of vaccines [19] against corona virus was certainly a milestone. However, on a world-wide basis, the shortage of vaccines in comparison to their huge demand necessitates the development of further medicaments against SARS-CoV-2.

SARS-CoV-2 is a respiratory virus that enters a host mainly through the nasal mucosa as the first entry point. The entry of SARS-CoV-2 into the host cells is primarily facilitated by the nonspecific electrostatic interaction between the heparan sulfates proteoglycans present on the host cells and the spike proteins on surface of the viruses. [40]

Recently, Wolf et al. [41] developed a novel ex *vivo* model system comprising of human nasal turbinate tissues to evaluate the efficiency of the synthetic inhibitors against respiratory viruses in the first stage of infection at the nasal mucosal entry site. Using this model system, they screened a series of chemical compounds to finalize the best one that would be useful in nasal spray, but they did not reveal the chemical structure of the most active compound in their studies. In this context, synthetic sulfates would be promising ingredients in nasal spray owing to their negatively charged sulfate groups that could interact with the surface proteins of SARS-CoV-2.

Recently, Haag et al. [24] reported polysulfate inhibitors, namely, a variety of structural analogues of sulfated polyglycerol such as linear polyglycerol sulfate (LPGS) and hyperbranched polyglycerol sulfate (HPGS). They concluded the LPGS as the best inhibitors.

LPGS showed SARS-CoV-2 inhibition with low half maximal inhibitory concentration (IC₅₀ ~ 66.9 µg/mL) which was -60 and -20 times lower than that observed for the natural polysulfates such as Heparin or pentosan sulfate, respectively. The superior activity of LPGS was explained by its flexible chain conformation that was considered to help interacting with the virus surface proteins more effectively compared to the other analogous.

It is an object of the present invention to provide SARS-CoV-2 inhibitors that are even more effective than LPGS. Another object is to provide appropriate manufacturing methods for such inhibitors.

This object is achieved with a polymer having a structure according to general formula (I):

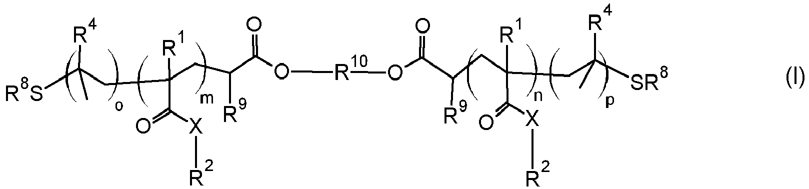

In this context, the residues have the following meanings:
- R¹ =: -CH₃ or -H,
- R² =: 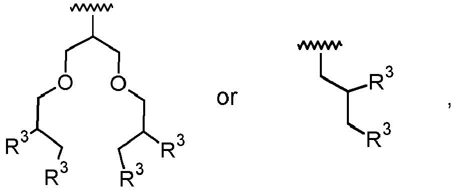
- R³ =: independently from other R³ residues in the same molecule: -OH, -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 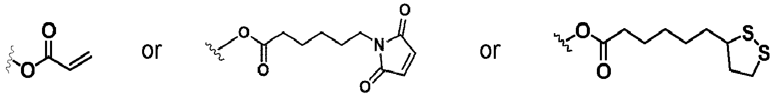 or a salt of any of these residues,
- R⁴ =: independently from other R⁴ residues in the same molecule: 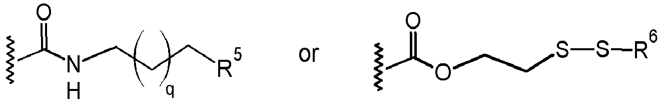 or 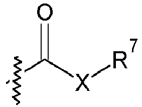
- R⁵ =: independently from other R⁵ residues in the same molecule: 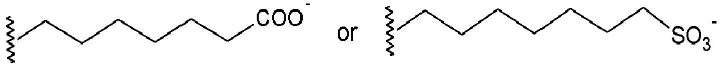 or 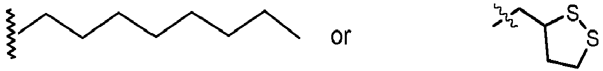 or 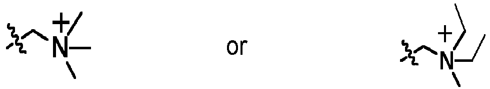 or a salt of any of these residues,
- R⁶ =: independently from other R⁶ residues in the same molecule: 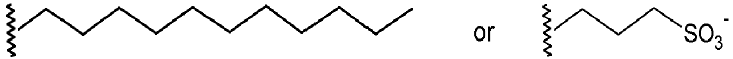 or 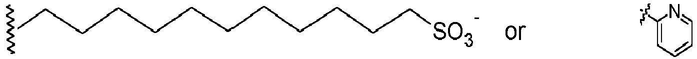 or 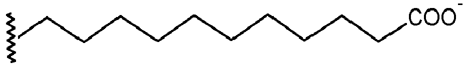 or a salt of any of these residues,
- R⁷ =: independently from other R⁷ residues in the same molecule: 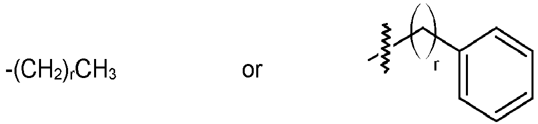 or 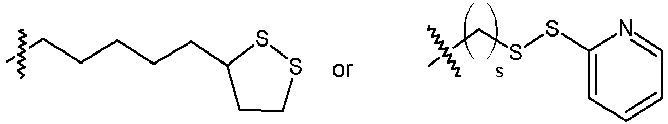 or 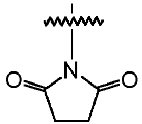
- R⁸ =: independently from other R⁸ residues in the same molecule: 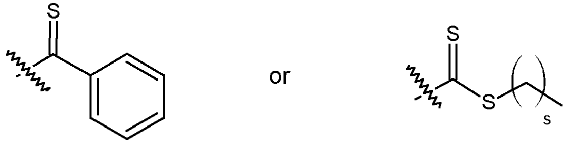 or 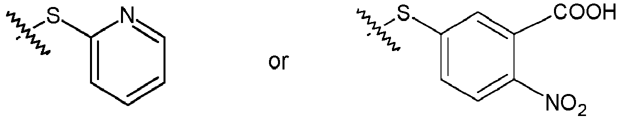 or 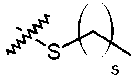
- R⁹ =: independently from other R⁹ residues in the same molecule: a methyl residue or a phenyl residue,
- R¹⁰ =: independently from other R¹⁰ residues in the same molecule: a substituted or non-substituted C₁-C₁₂ alkyl residue or a substituted or non-substituted C₆-C₁₂ aryl residue,
- X =: independently from other X residues in the same molecule: -O or -NH,
- m =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- n =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- o =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- p =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- q =: independently from other q indices in the same molecule: 1 to 10, in particular 2 to 9, in particular 3 to 8, in particular 4 to 7, in particular 5 to 6,
- r =: independently from other r indices in the same molecule: 0 to 11, in particular 1 to 10, in particular 2 to 9, in particular 3 to 8, in particular 4 to 7, in particular 5 to 6, and
- s =: independently from other s indices in the same molecule: 1 to 12, in particular 2 to 11, in particular 3 to 10, in particular 4 to 9, in particular 5 to 8, in particular 6 to 7.

Such a polymer, in particular when carrying a negatively charged residue like sulfate, sulfonate, phosphate and/or carboxylate (-OSO₃⁻, -O(CH₂)_{q}SO₃⁻, -(CH₂)_{q}SO₃⁻, -OPO₃²⁻, -OCO(CH₂)_{q}COO⁻), shows excellent binding capacity against SARS-CoV-2. To give a specific example, a relatively high molecular weight (-400 KDa) methacrylate based dendronized polyglycerol sulfate (pDenG1MAS, also referred to as PGMAS or P1) corresponding to general formula (I) showed an excellent SARS-CoV-2 inhibition with a remarkably low IC₅₀ value ~ 0.3 µg/mL which is 220 times lower than that observed for LPGS. Despite not having a flexible structure like LPGS, pDenG1MAS surprisingly showed better inhibition results which is contrary to the recently reported results from the inventors' group. This indicates that the chain flexibility is apparently not the only determining parameter for the potency of viral inhibitors.

Rather, the long fibers of pDenG1MAS may play an important role in virus inhibition. On the one hand, they could cover the sulfate binding domain of the SARS-CoV-2 to interact efficiently in a polyvalent fashion and on the other hand they could provide the steric shedding to restrict the virus to bind with the active receptor angiotensin-converting enzyme 2 (ACE2) on the cell surface. Therefore, probably because of this combining effect, pDenG1MAS appears to be a significantly better SARS-CoV-2 inhibitor than LPGS.

Besides, sulfated polymers sometimes show an anticoagulant activity that restricts their use in most clinical applications. In this context, like LPGS, pDenG1MAS also showed a remarkably (-10 times) lower anticoagulant activity than Heparin sulfates. This makes the polymers according to general formula (I) promising candidates for antiviral drugs. Easy synthesis in bulk scale, the remarkably low IC₅₀ values for SARS-CoV-2 inhibition and negligible anticoagulant activity allow to use polymers according to general formula (I) like pDenG1MAS as the pharmaceutically active ingredient in a nasal spray.

Though both acrylate [42] and methacrylate [43] based hydroxyl containing dendronized glycerol polymers have already been reported from the inventors' group, the structure of the polymers according to general formula (I) is significantly different from the reported structures. Apart from the relatively higher molecular weight compared to the compounds reported earlier, also the manufacturing method differs from the previously published manufacturing method.

The polymers according to general formula (I) can be synthesized using a bi-functional chain transfer agent that helps to introduce a 2-pyridyl disulfide at the both terminals of the polymer. This 2-pyridyl disulfide can be utilized to further grow the polymer chain from both terminals to synthesize a high molecular weight version of the polymer. This opportunity is not possible with the previously reported polymeric structures [42, 43]

In an embodiment, the (in particular pharmaceutically acceptable) salt is a sodium salt or a potassium salt. Sodium is a particularly appropriate counter ion for the negatively charged residues.

In an embodiment, X is -O if residue R⁷ is

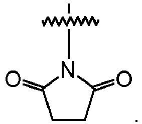

In an embodiment, a degree of functionalization of the polymer lies in a range of from 10 to 100 %, in particular from 11 % to 99 %, in particular from 12 % to 98 %, in particular from 15 % to 97 %, in particular from 16 % to 96 %, in particular from 17 % to 95 %, in particular from 18 % to 94 %, in particular from 19 % to 93 %, in particular from 20 % to 90 %, in particular from 30 % to 85 %, in particular from 40 % to 70 %, in particular from 50 % to 60 %, in particular from 70 % to 98 %, in particular from 75 % to 95 %, in particular from 80 % to 90 %. The degree of functionalization is defined by R³ residues that are different from hydroxyl groups. If all R³ residues are different from hydroxyl groups, the degree of functionalization is 100 %. If one half of the R³ residues are hydroxyl groups and the other half are different residues, the degree of functionalization is 50 %.

In an embodiment, R³ is independently from other R³ residues in the same molecule -OH, -OSO₃⁻, -O(CH₂)_{q}SO₃⁻, -(CH₂)_{q}SO₃⁻, -OPO₃²⁻, -OCO(CH₂)_{q}COO⁻ or a salt of any of these residues.

In an embodiment, o and p are 0. Then, general formula (I) is simplified to general formula (XVI):

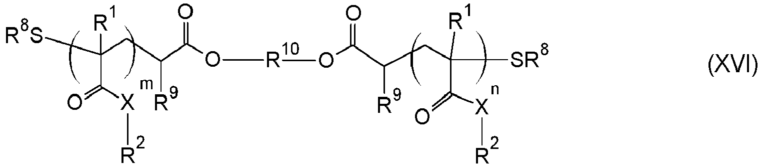

Examples of specific polymers covered by general formula (XVI) are summarized in the following Table 1, wherein residue R² is in each case

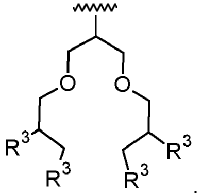

**Table 1: Polymers according to formula (XVI):**

| **Polymer** | **R¹** | **X** | **R³** | **Active against following viruses/bacteri** a | **m + n** |
|---|---|---|---|---|---|
| **P0** | -CH₃ | -O | -OSO₃Na | SARS-CoV-2, HSV-1, RSV | 20 to 1000 |
| **P1** | -CH₃ | -O | -OSO₃Na | SARS-CoV-2, HSV-1, RSV | 580 |
| **P2** | -CH₃ | -O | -OSO₃Na | SARS-CoV-2, HSV-1, RSV | 180 |
| **P3** | -CH₃ | -O | -OSO₃Na | SARS-CoV-2, HSV-1, RSV | 60 |
| **P4** | -CH₃ | -O | -OPO₃²⁻ | SARS-CoV-2, HSV-1, RSV | 20 to 1000 |
| **P5** | -H | -NH | -OPO₃²⁻ | SARS-CoV-2, HSV-1, RSV | 20 to 1000 |
| **P6** | -H | -NH | -OSO₃Na | SARS-CoV-2, HSV-1, RSV | 20 to 1000 |
| **P7** | -H | -NH | -OCOCH₂CH₂COONa | SARS-CoV-2, HSV-1, RSV | 20 to 1000 |
| **P7a** | -CH₃ | -O | -OCOCH₂CH₂COONa | SARS-CoV-2, HSV-1, RSV | 20 to 1000 |
| **P8** | -CH₃ | -O | Sialic acid | Influenza, in particular influenza A | 20 to 1000 |
| **P9** | -H | -NH | Sialic acid | Influenza, in particular influenza A | 20 to 1000 |
| **P10** | -CH₃ | -O | Fucose | Pseudomonas aeruginosa | 20 to 1000 |
| **P11** | -H | -NH | Fucose | Pseudomonas aeruginosa | 20 to 1000 |
| **P12** | -CH₃ | -O | Galactose | Pseudomonas aeruginosa | 20 to 1000 |
| **P13** | -H | -NH | Galactose | Pseudomonas aeruginosa | 20 to 1000 |
| **P14** | -CH₃ | -O | Mannose | E. Coli | 20 to 1000 |
| **P15** | -H | -NH | Mannose | E. Coli | 20 to 1000 |

Further examples of specific polymers covered by general formula (XVI) are summarized in the following Table 2, wherein residue R² is in each case

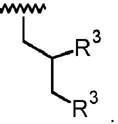

**Table 2: Polymers according to formula (XVI):**

| **Polymer** | **R¹** | **X** | **R³** | **Active against following viruses/bacteri a** | **m + n** |
|---|---|---|---|---|---|
| **P0_1** | -CH₃ | -O | -OSO₃Na | SARS-CoV-2, HSV-1, RSV | 20 to 1000 |
| **P1_1** | -CH₃ | -O | -OSO₃Na | SARS-CoV-2, HSV-1, RSV | 580 |
| **P2_1** | -CH₃ | -O | -OSO₃Na | SARS-CoV-2, HSV-1, RSV | 180 |
| **P43** | -CH₃ | -O | -OSO₃Na | SARS-CoV-2, HSV-1, RSV | 60 |
| **P44** | -CH₃ | -O | -OCOCH₂CH₂COONa | SARS-CoV-2, HSV-1, RSV | 20 to 1000 |
| **P45** | -CH₃ | -O | -OPO₃²⁻ | SARS-CoV-2, HSV-1, RSV | 20 to 1000 |
| **P46** | -H | -NH | -OPO₃²⁻ | SARS-CoV-2, HSV-1, RSV | 20 to 1000 |
| **P47** | -H | -NH | -OSO₃Na | SARS-CoV-2, HSV-1, RSV | 20 to 1000 |
| **P48** | -H | -NH | -OCOCH₂CH₂COONa | SARS-CoV-2, HSV-1, RSV | 20 to 1000 |
| **P49** | -CH₃ | -O | Sialic acid | Influenza, in particular influenza A | 20 to 1000 |
| **P50** | -H | -NH | Sialic acid | Influenza, in particular influenza A | 20 to 1000 |
| **P51** | -CH₃ | -O | Fucose | Pseudomonas aeruginosa | 20 to 1000 |
| **P52** | -H | -NH | Fucose | Pseudomonas aeruginosa | 20 to 1000 |
| **P53** | -CH₃ | -O | Galactose | Pseudomonas aeruginosa | 20 to 1000 |
| **P54** | -H | -NH | Galactose | Pseudomonas aeruginosa | 20 to 1000 |
| **P55** | -CH₃ | -O | Mannose | E. Coli | 20 to 1000 |
| **P56** | -H | -NH | Mannose | E. Coli | 20 to 1000 |

In an embodiment, m and n are equally big.

In an embodiment, a sum of m and n lies in a range of from 50 to 700, in particular from 60 to 750, in particular from 70 to 700, in particular from 80 to 650, in particular from 90 to 600, in particular from 100 to 550, in particular from 150 to 500, in particular from 200 to 450, in particular from 250 to 350.

In an embodiment, X is O.

Examples of specific polymers covered by general formula (I) are summarized in the following Table 3, wherein X is O, residue R¹ is -CH₃, residue R² is 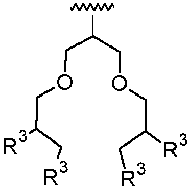 and residue R⁴ is

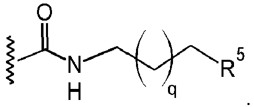

**Table 3: Polymers according to formula (I):**

| **Polymer** | **R³** | **R⁵** | **Active against following viruses/bacteri a** | **m + n** |
|---|---|---|---|---|
| **P16** | -OSO₃Na | 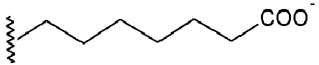 | SARS-CoV-2, HSV-1, RSV | 20 to 1000 |
| **P17** | -OSO₃Na | 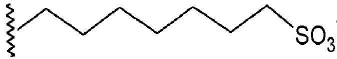 | SARS-CoV-2, HSV-1, RSV | 20 to 1000 |
| **P18** | -OSO₃Na | 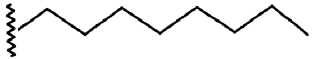 | SARS-CoV-2, HSV-1, RSV | 20 to 1000 |
| **P19** | -OSO₃Na, Sialic acid | 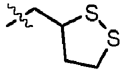 | SARS-CoV-2, HSV-1, RSV, Influenza, in particular influenza A | 20 to 1000 |
| **P20** | -OH | 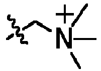 | E. coli | 20 to 1000 |
| **P21** | -OH | 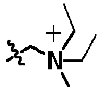 | E. coli | 20 to 1000 |

Examples of specific polymers covered by general formula (I) are summarized in the following Table 4, wherein X is O, residue R¹ is -CH₃, residue R² is 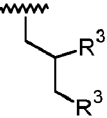 and residue R⁴ is

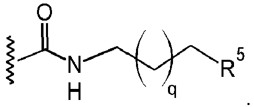

**Table 4: Polymers according to formula (I):**

| **Polymer** | **R³** | **R⁵** | **Active against following viruses/bacteri** a | **m + n** |
|---|---|---|---|---|
| **P16_1** | -OSO₃Na | 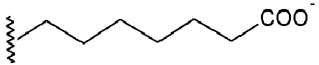 | SARS-CoV-2, HSV-1, RSV | 20 to 1000 |
| **P17_1** | -OSO₃Na | 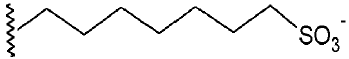 | SARS-CoV-2, HSV-1, RSV | 20 to 1000 |
| **P18_1** | -OSO₃Na | 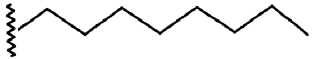 | SARS-CoV-2, HSV-1, RSV | 20 to 1000 |
| **P19_1** | -OSO₃Na, Sialic acid | 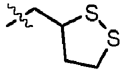 | SARS-CoV-2, HSV-1, RSV, Influenza, in particular influenza A | 20 to 1000 |
| **P20_1** | -OH | 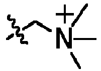 | E. coli | 20 to 1000 |
| **P21_1** | -OH | 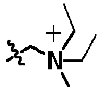 | E. coli | 20 to 1000 |

Examples of specific polymers covered by general formula (I) are summarized in the following Table 5, wherein X is O, residue R¹ is -CH₃, residue R² is 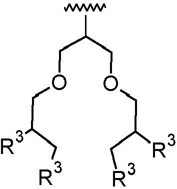 and residue R⁴ is

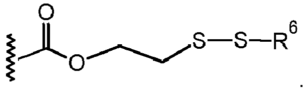

**Table 5: Polymers according to formula (I):**

| **Polymer** | **R³** | **R⁶** | **Active against following viruses/bacteri** a | **m + n** |
|---|---|---|---|---|
| **P22** | -OSO₃Na | 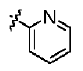 | SARS-CoV-2, HSV-1, RSV | 20 to 1000 |
| **P23** | -OSO₃Na, | 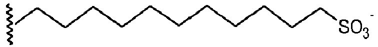 | SARS-CoV-2, HSV-1, RSV | 20 to 1000 |
| **P24** | -OSO₃Na | 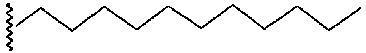 | SARS-CoV-2, HSV-1, RSV | 20 to 1000 |
| **P25** | -OSO₃Na | 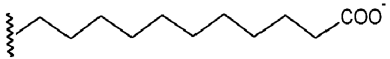 | SARS-CoV-2, HSV-1, RSV | 20 to 1000 |
| **P26** | -OSO₃Na | 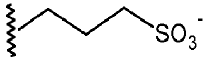 | SARS-CoV-2, HSV-1, RSV | 20 to 1000 |
| **P27** | Sialic acid | 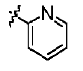 | Influenza, in particular influenza A | 20 to 1000 |
| **P28** | Mannose | 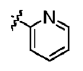 | E. coli | 20 to 1000 |
| **P29** | Fucose | 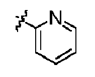 | Pseudomonas aeruginosa | 20 to 1000 |
| **P30** | Galactose | 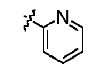 | Pseudomonas aeruginosa | 20 to 1000 |

Examples of specific polymers covered by general formula (I) are summarized in the following Table 6, wherein X is O, residue R¹ is -CH₃, residue R² is 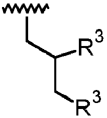 and residue R⁴ is

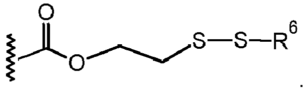

**Table 6: Polymers according to formula (I):**

| **Polymer** | **R³** | **R⁶** | **Active against following viruses/bacteri** a | **m + n** |
|---|---|---|---|---|
| **P22_1** | -OSO₃Na | 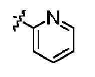 | SARS-CoV-2, HSV-1, RSV | 20 to 1000 |
| **P23_1** | -OSO₃Na, | 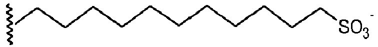 | SARS-CoV-2, HSV-1, RSV | 20 to 1000 |
| **P24_1** | -OSO₃Na | 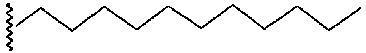 | SARS-CoV-2, HSV-1, RSV | 20 to 1000 |
| **P25_1** | -OSO₃Na | 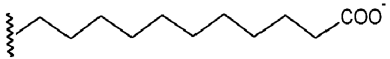 | SARS-CoV-2, HSV-1, RSV | 20 to 1000 |
| **P26_1** | -OSO₃Na | 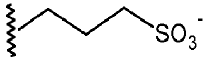 | SARS-CoV-2, HSV-1, RSV | 20 to 1000 |
| **P27_1** | Sialic acid | 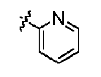 | Influenza, in particular influenza A | 20 to 1000 |
| **P28_1** | Mannose | 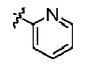 | E. coli | 20 to 1000 |
| **P29_1** | Fucose | 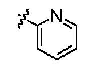 | Pseudomonas aeruginosa | 20 to 1000 |
| **P30_1** | Galactose | 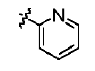 | Pseudomonas aeruginosa | 20 to 1000 |

In an aspect, the present invention relates to a hydrogel that is made from a polymer according to the preceding explanations. Such a hydrogel can be obtained by crosslinking such a polymer by ultraviolet light (e.g., having a wavelength lying in a range of from 250 nm to 400 nm, in particular from 275 nm to 375 nm, in particular from 300 nm to 365 nm, in particular from 325 nm to 350 nm) or with a crosslinker chosen from the group consisting of a PEG-dithiol (HS-PEG-SH), a 4-arm PEG-tetrathiol, and a PEG-dicyclooctyne corresponding to the following formula:

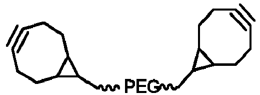

In an embodiment, the 4-arm PEG-tetrathiol corresponds to the following general formula (XIX):

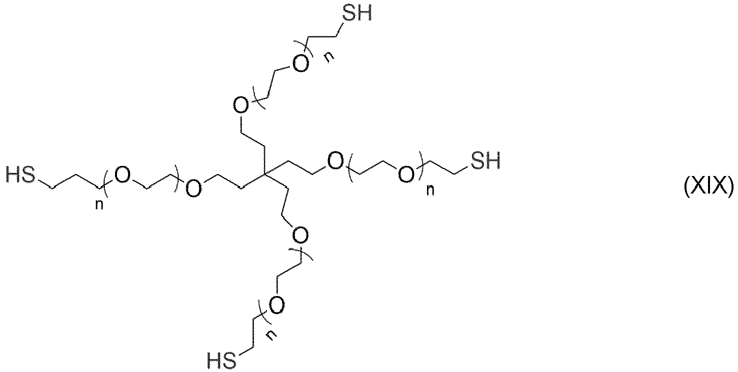

In this context, n lies in a range of from 10 to 100, in particular 20 to 90, in particular 30 to 80, in particular 40 to 70, in particular 50 to 60.

Examples of specific polymers covered by general formula (XVI) that are particularly appropriate for forming hydrogels are summarized in the following Table 7, wherein residue R² is in each case 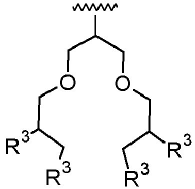 and wherein m + n is in each case 20 to 1000. Table 7 further lists appropriate crosslinkers for forming a hydrogel.

**Table 7: Polymers according to formula (XVI) that are particularly appropriate for forming hydrogels:**

| **Polymer** | **R¹** | **X** | **R³** | **Crosslinker for** hydrogel **formation** | **Active against following viruses/ bacteria** |
|---|---|---|---|---|---|
| **P31a** | -CH₃ | -O | 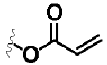 -OSO₃Na and | HS-PEG-HS | SARS-CoV-2, HSV-1, RSV |
| **P31b** | -H | -O | 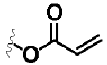 -OSO₃Na and | HS-PEG-HS | SARS-CoV-2, HSV-1, RSV |
| **P31c** | -CH₃ | -O | 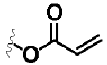 Sialic acid and | HS-PEG-HS | Influenza, in particular influenza A |
| **P31d** | -H | -O | 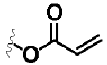 Sialic acid and | HS-PEG-HS | Influenza, in particular influenza A |
| **P31e** | -CH₃ | -NH | -OSO₃Na and 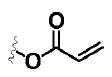 | HS-PEG-HS | SARS-CoV-2, HSV-1, RSV |
| **P31f** | -H | -NH | 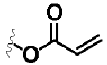 -OSO₃Na and | HS-PEG-HS | SARS-CoV-2, HSV-1, RSV |
| **P31g** | -CH₃ | -NH | 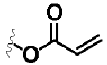 Sialic acid and | HS-PEG-HS | Influenza, in particular influenza A |
| **P31h** | -H | -NH | 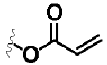 Sialic acid and | HS-PEG-HS | Influenza, in particular influenza A |
| **P32a** | -CH₃ | -O | 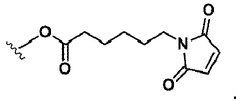 -OSO₃Na and | HS-PEG-HS | SARS-CoV-2, HSV-1, RSV |
| **P32b** | -H | -O | 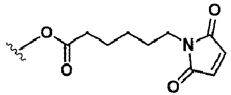 -OSO₃Na and | HS-PEG-HS | SARS-CoV-2, HSV-1, RSV |
| **P32c** | -CH₃ | -O | 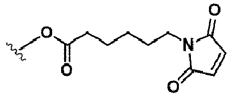 Sialic acid and | HS-PEG-HS | Influenza, in particular influenza A |
| **P32d** | -H | -O | 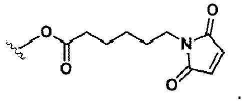 Sialic acid and | HS-PEG-HS | Influenza, in particular influenza A |
| **P32e** | -CH₃ | -NH | 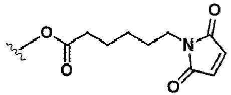 -OSO₃Na and | HS-PEG-HS | SARS-CoV-2, HSV-1, RSV |
| **P32f** | -H | -NH | 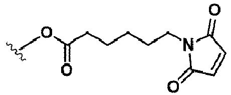 -OSO₃Na and | HS-PEG-HS | SARS-CoV-2, HSV-1, RSV |
| **P32g** | -CH₃ | -NH | 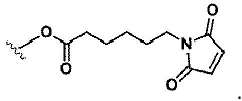 Sialic acid and | HS-PEG-HS | Influenza, in particular influenza A |
| **P32h** | -H | -NH | 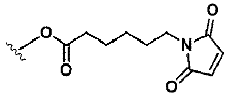 Sialic acid and | HS-PEG-HS | Influenza, in particular influenza A |
| **P33a** | -CH₃ | -O | 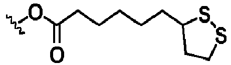 -OSO₃Na and | UV light (ca. 365 nm) | SARS-CoV-2, HSV-1, RSV |
| **P33b** | -H | -O | 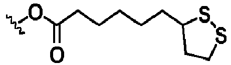 -OSO₃Na and | UV light (ca. 365 nm) | SARS-CoV-2, HSV-1, RSV |
| **P33c** | -CH₃ | -O | 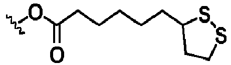 Sialic acid and | UV light (ca. 365 nm) | Influenza, in particular influenza A |
| **P33d** | -H | -O | 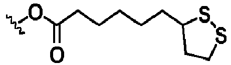 Sialic acid and | UV light (ca. 365 nm) | Influenza, in particular influenza A |
| **P33e** | -CH₃ | -NH | 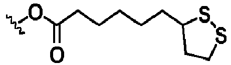 -OSO₃Na and | UV light (ca. 365 nm) | SARS-CoV-2, HSV-1, RSV |
| **P33f** | -H | -NH | 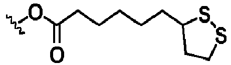 -OSO₃Na and | UV light (ca. 365 nm) | SARS-CoV-2, HSV-1, RSV |
| **P33g** | -CH₃ | -NH | 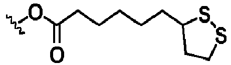 Sialic acid and | UV light (ca. 365 nm) | Influenza, in particular influenza A |
| **P33h** | -H | -NH | 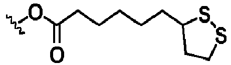 Sialic acid and | UV light (ca. 365 nm) | Influenza, in particular influenza A |
| **P34a** | -CH₃ | -O | 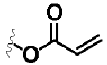 Fucose and | HS-PEG-HS | P. aeruginosa |
| **P34b** | -H | -O | 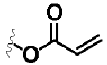 Fucose and | HS-PEG-HS | P. aeruginosa |
| **P34c** | -CH₃ | -O | 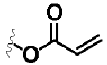 Galactose and | HS-PEG-HS | P. aeruginosa |
| **P34d** | -H | -O | 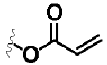 Galactose and | HS-PEG-HS | P. aeruginosa |
| **P34e** | -CH₃ | -NH | 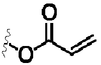 Fucose and | HS-PEG-HS | P. aeruginosa |
| **P34f** | -H | -NH | 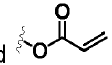 Fucose anc | HS-PEG-HS | P. aeruginosa |
| **P34g** | -CH₃ | -NH | 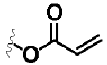 Galactose and | HS-PEG-HS | P. aeruginosa |
| **P34h** | -H | -NH | 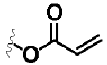 Galactose and | HS-PEG-HS | P. aeruginosa |
| **P35a** | -CH₃ | -O | 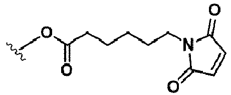 Fucose and | HS-PEG-HS | P. aeruginosa |
| **P35b** | -H | -O | 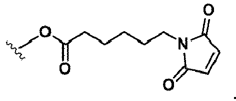 Fucose and | HS-PEG-HS | P. aeruginosa |
| **P35c** | -CH₃ | -O | 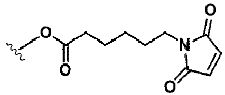 Galactose and | HS-PEG-HS | P. aeruginosa |
| **P35d** | -H | -O | 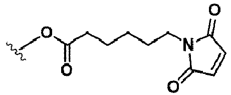 Galactose and | HS-PEG-HS | P. aeruginosa |
| **P35e** | -CH₃ | -NH | 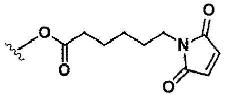 Fucose and | HS-PEG-HS | P. aeruginosa |
| **P35f** | -H | -NH | 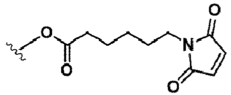 Fucose and | HS-PEG-HS | P. aeruginosa |
| **P35g** | -CH₃ | -NH | 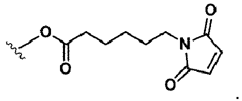 Galactose and | HS-PEG-HS | P. aeruginosa |
| **P35h** | -H | -NH | 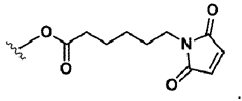 Galactose and | HS-PEG-HS | P. aeruginosa |
| **P36a** | -CH₃ | -O | 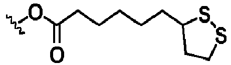 Fucose and | UV light (ca. 365 nm) | P. aeruginosa |
| **P36b** | -H | -O | 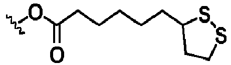 Fucose and | UV light (ca. 365 nm) | P. aeruginosa |
| **P36c** | -CH₃ | -O | 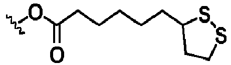 Galactose and | UV light (ca. 365 nm) | P. aeruginosa |
| **P36d** | -H | -O | 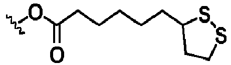 Galactose and | UV light (ca. 365 nm) | P. aeruginosa |
| **P36e** | -CH₃ | -NH | 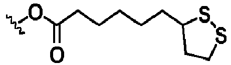 Fucose and | UV light (ca. 365 nm) | P. aeruginosa |
| **P36f** | -H | -NH | 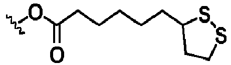 Fucose and | UV light (ca. 365 nm) | P. aeruginosa |
| **P36g** | -CH₃ | -NH | 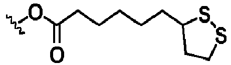 Galactose and | UV light (ca. 365 nm) | P. aeruginosa |
| **P36h** | -H | -NH | 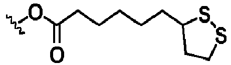 Galactose and | UV light (ca. 365 nm) | P. aeruginosa |
| **P37a** | -CH₃ | -O | 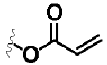 Mannose and | HS-PEG-HS | E. coli |
| **P37b** | -H | -O | 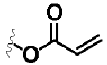 Mannose and | HS-PEG-HS | E. coli |
| **P37c** | -CH₃ | -NH | 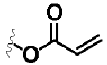 Mannose and | HS-PEG-HS | E. coli |
| **P37d** | -H | -NH | 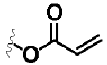 Mannose and | HS-PEG-HS | E. coli |
| **P38a** | -CH₃ | -O | 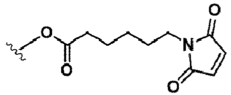 Mannose and | HS-PEG-HS | E. coli |
| P38b | -H | -O | 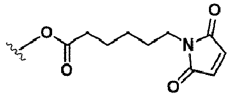 Mannose and | HS-PEG-HS | E. coli |
| **P38c** | -CH₃ | -NH | 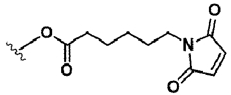 Mannose and | HS-PEG-HS | E. coli |
| P38d | -H | -NH | 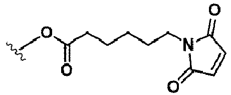 Mannose and | HS-PEG-HS | E. coli |
| **P39a** | -CH₃ | -O | 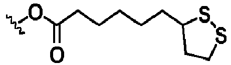 Mannose and | UV light (ca. 365 nm) | E. coli |
| **P39b** | -H | -O | 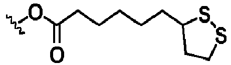 Mannose and | UV light (ca. 365 nm) | E. coli |
| **P39c** | -CH₃ | -NH | 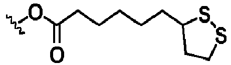 Mannose and | UV light (ca. 365 nm) | E. coli |
| **P39d** | -H | -NH | 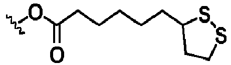 Mannose and | UV light (ca. 365 nm) | E. coli |
| **P40a** | -CH₃ | -O | -OSO₃Na and -N₃ | 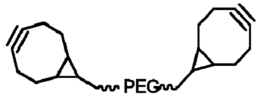 | SARS-CoV-2, HSV-1, RSV |
| **P40b** | -H | -O | -OSO₃Na and -N₃ | 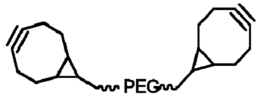 | SARS-CoV-2, HSV-1, RSV |
| **P40c** | -CH₃ | -O | Sialic acid and -N₃ | 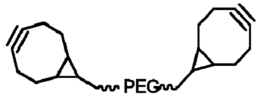 | Influenza, in particular influenza A |
| **P40d** | -H | -O | Sialic acid and -N₃ | 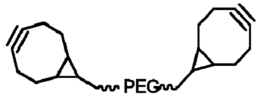 | Influenza, in particular influenza A |
| **P40e** | -CH₃ | -NH | -OSO₃Na and -N₃ | 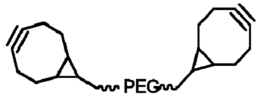 | SARS-CoV-2, HSV-1, RSV |
| **P40f** | -H | -NH | -OSO₃Na and -N₃ | 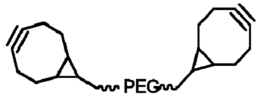 | SARS-CoV-2, HSV-1, RSV |
| **P40g** | -CH₃ | -NH | Sialic acid and -N₃ | 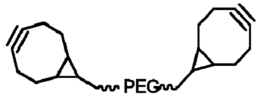 | Influenza, in particular influenza A |
| **P40h** | -H | -NH | Sialic acid and -N₃ | 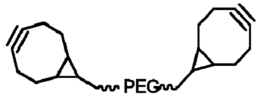 | Influenza, in particular influenza A |
| **P41a** | -CH₃ | -O | Fucose and -N₃ | 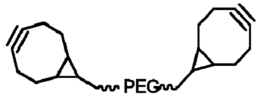 | P. aeruginosa |
| **P41b** | -H | -O | Fucose and -N₃ | 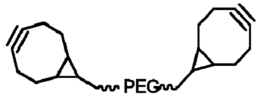 | P. aeruginosa |
| **P41c** | -CH₃ | -O | Galactose and -N₃ | 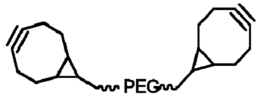 | P. aeruginosa |
| **P41d** | -H | -O | Galactose and -N₃ | 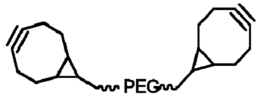 | P. aeruginosa |
| **P41e** | -CH₃ | -NH | Fucose and -N₃ | 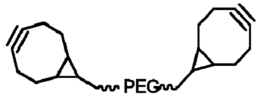 | P. aeruginosa |
| **P41f** | -H | -NH | Fucose and -N₃ | 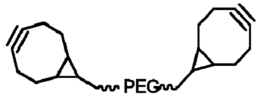 | P. aeruginosa |
| **P41g** | -CH₃ | -NH | Galactose and -N₃ | 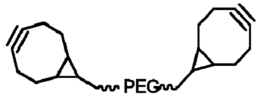 | P. aeruginosa |
| **P41h** | -H | -NH | Galactose and -N₃ | 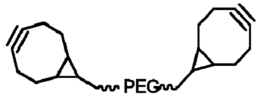 | P. aeruginosa |
| P42a | -CH₃ | -O | Mannose and -N₃ | 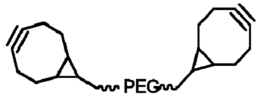 | E. coli |
| P42b | -H | -O | Mannose and -N₃ | 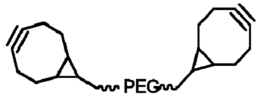 | E. coli |
| P42c | -CH₃ | -NH | Mannose and -N₃ | 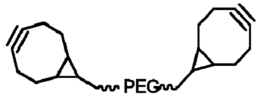 | E. coli |
| P42d | -H | -NH | Mannose and -N₃ | 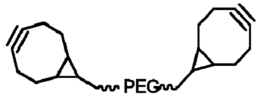 | E. coli |

Further Examples of specific polymers covered by general formula (XVI) that are particularly appropriate for forming hydrogels are summarized in the following Table 8, wherein residue R² is in each case 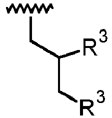 and wherein m + n is in each case 20 to 1000. Table 8 further lists appropriate crosslinkers for forming a hydrogel.

**Table 8: Polymers according to formula (XVI) that are particularly appropriate for forming hydrogels:**

| Polymer | **R¹** | X | **R³** | Crosslinker for hydrogel formation | Active against following **viruses/** bacteria |
|---|---|---|---|---|---|
| **P31a** _1 | -CH₃ | -O | 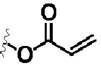 -OSO₃Na and | HS-PEG-HS | SARS-CoV-2, HSV-1, RSV |
| **P31b _1** | -H | -O | 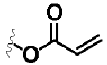 -OSO₃Na and | HS-PEG-HS | SARS-CoV-2, HSV-1, RSV |
| **P31c _1** | -CH₃ | -O | 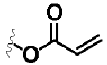 Sialic acid and | HS-PEG-HS | Influenza, in particular influenza A |
| **P31d _1** | -H | -O | 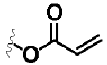 Sialic acid and | HS-PEG-HS | Influenza, in particular influenza A |
| **P31e _1** | -CH₃ | -NH | 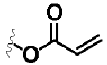 -OSO₃Na and | HS-PEG-HS | SARS-CoV-2, HSV-1, RSV |
| P31f **_1** | -H | -NH | 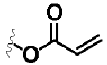 -OSO₃Na and | HS-PEG-HS | SARS-CoV-2, HSV-1, RSV |
| **P31g _1** | -CH₃ | -NH | 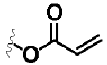 Sialic acid and | HS-PEG-HS | Influenza, in particular influenza A |
| **P31h _1** | -H | -NH | 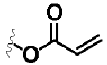 Sialic acid and | HS-PEG-HS | Influenza, in particular influenza A |
| P32a **_1** | -CH₃ | -O | 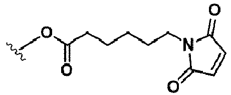 -OSO₃Na and | HS-PEG-HS | SARS-CoV-2, HSV-1, RSV |
| **P32b _1** | -H | -O | 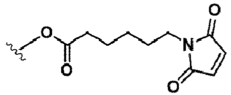 -OSO₃Na and | HS-PEG-HS | SARS-CoV-2, HSV-1, RSV |
| **P32c _1** | -CH₃ | -O | 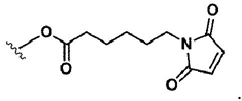 Sialic acid and | HS-PEG-HS | Influenza, in particular influenza A |
| P32d **_1** | -H | -O | 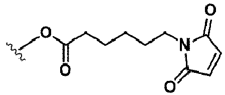 Sialic acid and | HS-PEG-HS | Influenza, in particular influenza A |
| **P32e _1** | -CH₃ | -NH | 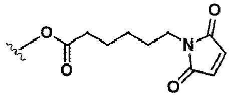 -OSO₃Na and | HS-PEG-HS | SARS-CoV-2, HSV-1, RSV |
| **P32f _1** | -H | -NH | 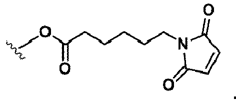 -OSO₃Na and | HS-PEG-HS | SARS-CoV-2, HSV-1, RSV |
| **P32g _1** | -CH₃ | -NH | 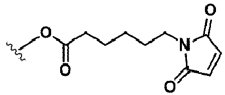 Sialic acid and | HS-PEG-HS | Influenza, in particular influenza A |
| P32h **_1** | -H | -NH | 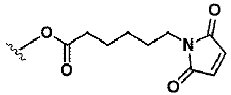 Sialic acid and | HS-PEG-HS | Influenza, in particular influenza A |
| **P33a _1** | -CH₃ | -O | 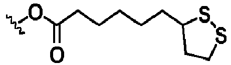 -OSO₃Na and | UV light (ca. 365 nm) | SARS-CoV-2, HSV-1, RSV |
| **P33b _1** | -H | -O | 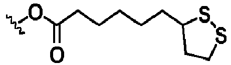 -OSO₃Na and | UV light (ca. 365 nm) | SARS-CoV-2, HSV-1, RSV |
| **P33c _1** | -CH₃ | -O | 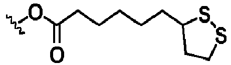 Sialic acid and | UV light (ca. 365 nm) | Influenza, in particular influenza A |
| **P33d _1** | -H | -O | 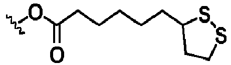 Sialic acid and | UV light (ca. 365 nm) | Influenza, in particular influenza A |
| **P33e _1** | -CH₃ | -NH | 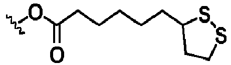 -OSO₃Na and | UV light (ca. 365 nm) | SARS-CoV-2, HSV-1, RSV |
| P33f **_1** | -H | -NH | 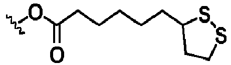 -OSO₃Na and | UV light (ca. 365 nm) | SARS-CoV-2, HSV-1, RSV |
| **P33g _1** | -CH₃ | -NH | 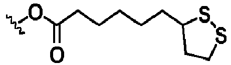 Sialic acid and | UV light (ca. 365 nm) | Influenza, in particular influenza A |
| **P33h _1** | -H | -NH | 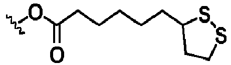 Sialic acid and | UV light (ca. 365 nm) | Influenza, in particular influenza A |
| **P34a _1** | -CH₃ | -O | 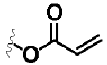 Fucose and | HS-PEG-HS | P. aeruginosa |
| **P34b _1** | -H | -O | 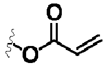 Fucose and | HS-PEG-HS | P. aeruginosa |
| **P34c _1** | -CH₃ | -O | 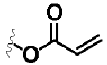 Galactose and | HS-PEG-HS | P. aeruginosa |
| **P34d _1** | -H | -O | 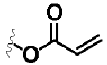 Galactose and | HS-PEG-HS | P. aeruginosa |
| **P34e _1** | -CH₃ | -NH | 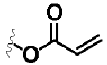 Fucose and | HS-PEG-HS | P. aeruginosa |
| **P34f _1** | -H | -NH | 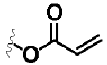 Fucose and | HS-PEG-HS | P. aeruginosa |
| P34g **_1** | -CH₃ | -NH | 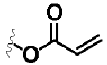 Galactose and | HS-PEG-HS | P. aeruginosa |
| **P34h _1** | -H | -NH | 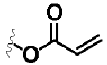 Galactose and | HS-PEG-HS | P. aeruginosa |
| **P35a _1** | -CH₃ | -O | 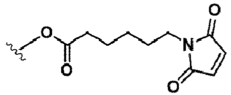 Fucose and | HS-PEG-HS | P. aeruginosa |
| **P35b _1** | -H | -O | 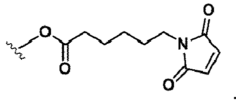 Fucose and | HS-PEG-HS | P. aeruginosa |
| **P35c _1** | -CH₃ | -O | 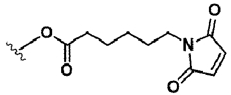 Galactose and | HS-PEG-HS | P. aeruginosa |
| **P35d _1** | -H | -O | 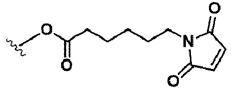 Galactose and | HS-PEG-HS | P. aeruginosa |
| **P35e _1** | -CH₃ | -NH | 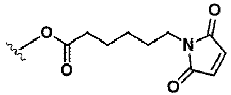 Fucose and | HS-PEG-HS | P. aeruginosa |
| **P35f _1** | -H | -NH | 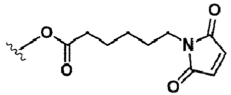 Fucose and | HS-PEG-HS | P. aeruginosa |
| **P35g _1** | -CH₃ | -NH | 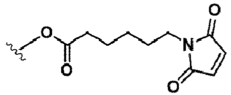 Galactose and | HS-PEG-HS | P. aeruginosa |
| **P35h _1** | -H | -NH | 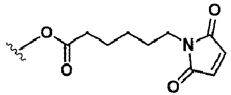 Galactose and | HS-PEG-HS | P. aeruginosa |
| **P36a _1** | -CH₃ | -O | 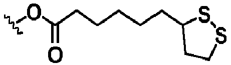 Fucose and | UV light (ca. 365 nm) | P. aeruginosa |
| P36b _1 | -H | -O | 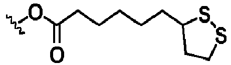 Fucose and | UV light (ca. 365 nm) | P. aeruginosa |
| P36c _1 | -CH₃ | -O | 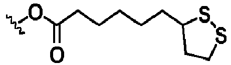 Galactose and | UV light (ca. 365 nm) | P. aeruginosa |
| P36d _1 | -H | -O | 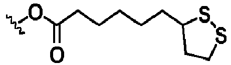 Galactose and | UV light (ca. 365 nm) | P. aeruginosa |
| **P36e _1** | -CH₃ | -NH | 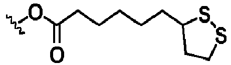 Fucose and | UV light (ca. 365 nm) | P. aeruginosa |
| **P36f _1** | -H | -NH | 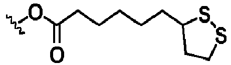 Fucose and | UV light (ca. 365 nm) | P. aeruginosa |
| **P36g _1** | -CH₃ | -NH | 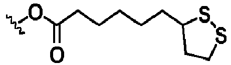 Galactose and | UV light (ca. 365 nm) | P. aeruginosa |
| **P36h _1** | -H | -NH | 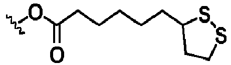 Galactose and | UV light (ca. 365 nm) | P. aeruginosa |
| **P37a _1** | -CH₃ | -O | 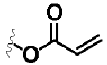 Mannose and | HS-PEG-HS | E. coli |
| **P37b _1** | -H | -O | 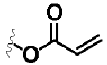 Mannose and | HS-PEG-HS | E. coli |
| **P37c _1** | -CH₃ | -NH | 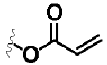 Mannose and | HS-PEG-HS | E. coli |
| **P37d _1** | -H | -NH | 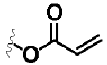 Mannose and | HS-PEG-HS | E. coli |
| **P38a _1** | -CH₃ | -O | 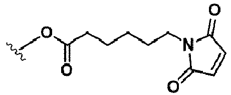 Mannose and | HS-PEG-HS | E. coli |
| **P38b _1** | -H | -O | 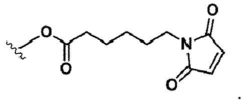 Mannose and | HS-PEG-HS | E. coli |
| **P38c _1** | -CH₃ | -NH | 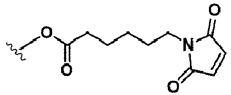 Mannose and | HS-PEG-HS | E. coli |
| **P38d _1** | -H | -NH | 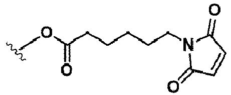 Mannose and | HS-PEG-HS | E. coli |
| **P39a _1** | -CH₃ | -O | 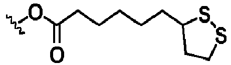 Mannose and | UV light (ca. 365 nm) | E. coli |
| **P39b _1** | -H | -O | 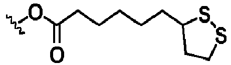 Mannose and | UV light (ca. 365 nm) | E. coli |
| **P39c _1** | -CH₃ | -NH | 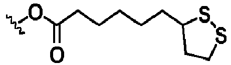 Mannose and | UV light (ca. 365 nm) | E. coli |
| P39d **_1** | -H | -NH | 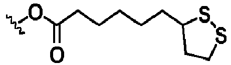 Mannose and | UV light (ca. 365 nm) | E. coli |
| P40a **_1** | -CH₃ | -O | -OSO₃Na and -N₃ | 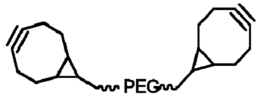 | SARS-CoV-2, HSV-1, RSV |
| **P40b _1** | -H | -O | -OSO₃Na and -N₃ | 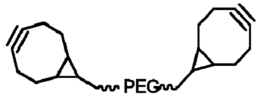 | SARS-CoV-2, HSV-1, RSV |
| **P40c _1** | -CH₃ | -O | Sialic acid and -N₃ | 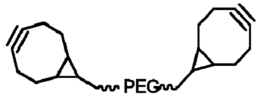 | Influenza, in particular influenza A |
| **P40d _1** | -H | -O | Sialic acid and -N₃ | 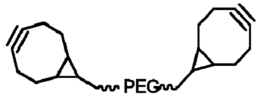 | Influenza, in particular influenza A |
| **P40e _1** | -CH₃ | -NH | -OSO₃Na and -N₃ | 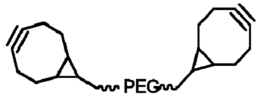 | SARS-CoV-2, HSV-1, RSV |
| **P40f _1** | -H | -NH | -OSO₃Na and -N₃ | 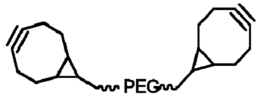 | SARS-CoV-2, HSV-1, RSV |
| **P40g _1** | -CH₃ | -NH | Sialic acid and -N₃ | 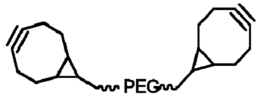 | Influenza, in particular influenza A |
| **P40h _1** | -H | -NH | Sialic acid and -N₃ | 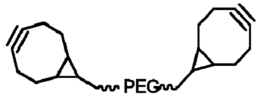 | Influenza, in particular influenza A |
| **P41a _1** | -CH₃ | -O | Fucose and -N₃ | 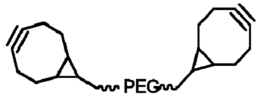 | P. aeruginosa |
| **P41b _1** | -H | -O | Fucose and -N₃ | 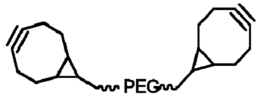 | P. aeruginosa |
| **P41c _1** | -CH₃ | -O | Galactose and -N₃ | 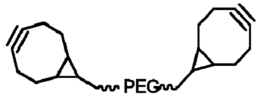 | P. aeruginosa |
| **P41d _1** | -H | -O | Galactose and -N₃ | 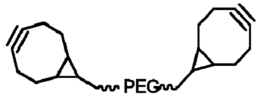 | P. aeruginosa |
| **P41e _1** | -CH₃ | -NH | Fucose and -N₃ | 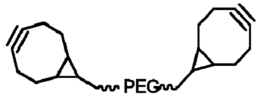 | P. aeruginosa |
| **P41f _1** | -H | -NH | Fucose and -N₃ | 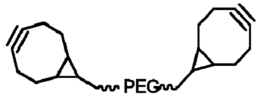 | P. aeruginosa |
| **P41g _1** | -CH₃ | -NH | Galactose and -N₃ | 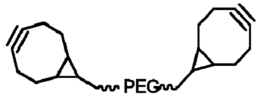 | P. aeruginosa |
| **P41h _1** | -H | -NH | Galactose and -N₃ | 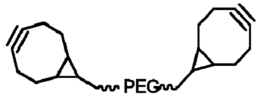 | P. aeruginosa |
| **P42a _1** | -CH₃ | -O | Mannose and -N₃ | 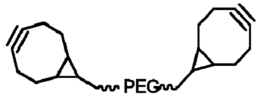 | E. coli |
| **P42b _1** | -H | -O | Mannose and -N₃ | 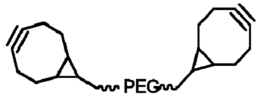 | E. coli |
| P42c _1 | -CH₃ | -NH | Mannose and -N₃ | 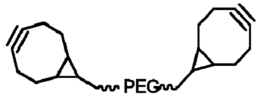 | E. coli |
| **P42d** _1 | -H | -NH | Mannose and -N₃ | 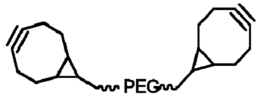 | E. coli |

In an aspect, the present invention relates to the in vivo or in vitro use of a polymer or a hydrogel according to the preceding explanations for binding viruses and/or bacteria.

In an embodiment, the viruses are chosen from the group consisting of influenza viruses (e.g., influenza A), coronaviruses (e.g., SARS-CoV-2), herpes simplex viruses (HSV, e.g., HSV-1), and respiratory syncytial viruses (RSV), and/or in that the bacteria are chosen from the group consisting of pseudomonas (e.g., P. aeruginosa) and Escherichia coli (E. coli).

In an aspect, the present invention relates to the medical use of a polymer or a hydrogel according to the preceding explanations in antiviral and/or antibacterial therapy of humans or animals.

In an aspect, the present invention relates to an antiviral and/or antibacterial method of treatment of a human or animal patient in need thereof. This method comprises a step of administering a polymer or a hydrogel according to the preceding explanations in a pharmaceutically active amount to a patient in need thereof. The administering can be done topically or systemically. In an embodiment, the administering is done topically, e.g., by applying the polymer or hydrogel in form of a nasal spray.

In an aspect, the present invention relates to the medical use of a polymer or a hydrogel according to the preceding explanations as mucus replacement, as cartilage replacement, or as synovial fluid replacement in a patient in need thereof. The rheological and viscoelastic properties of the hydrogel can be finely tuned to match the corresponding properties of mucus, cartilage, or synovial fluid. This opens a wide scope of applications of the presently claimed and described polymer and hydrogel. In addition, the respective mucus replacement, cartilage replacement, or synovial fluid replacement exhibits antiviral/antibacterial properties and can thus prevent infections and assist healing processes.

In an aspect, the present invention relates to the use of a polymer or a hydrogel according to the preceding explanations as matrix for cultivating and/or expanding cells and/or organoids in vitro. Particularly appropriate cells for such cell culture/expansion technique are stem cells.

In an aspect, the present invention relates to a pharmaceutical composition comprising a polymer or a hydrogel according to the preceding explanations. An appropriate dosage form of this pharmaceutical composition is a nasal spray.

In an aspect, the present invention relates to the use of a polymer or a hydrogel according to the preceding explanations as lubricant. A polymer in which all residues R³ are non-substituted hydroxyl groups is particularly appropriate for such use as lubricant.

In an aspect, the present invention relates to a first method for manufacturing a polymer according to the preceding explanations. This method comprises the steps explained in the following.

In a method step, a compound according to general formula (II) is reacted with a compound according to general formula (III) to obtain a compound according to general formula (IV):

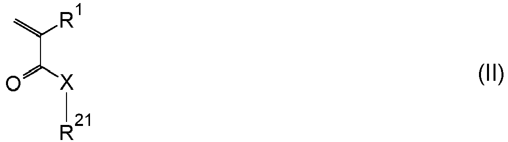

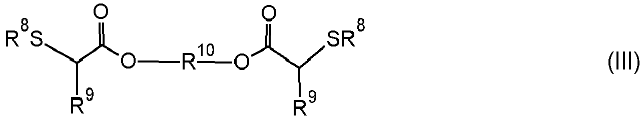

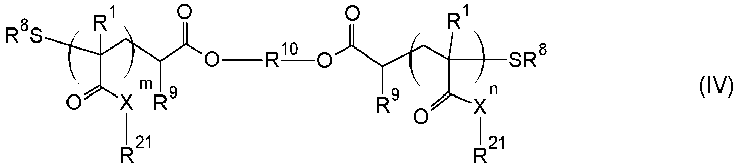

In this context,
- R¹ =: -CH₃ or -H,
- R⁸ =: independently from other R⁸ residues in the same molecule: 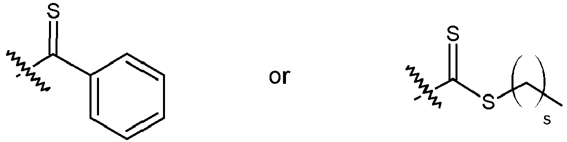 or 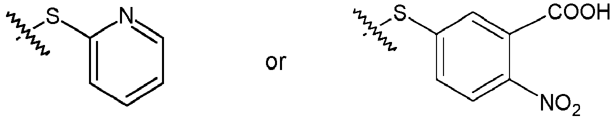 or 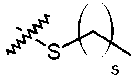
- R⁹ =: independently from other R⁹ residues in the same molecule: a methyl residue or a phenyl residue,
- R¹⁰ =: independently from other R¹⁰ residues in the same molecule: a substituted or non-substituted C₁-C₁₂ alkyl residue or a substituted or non-substituted C₆-C₁₂ aryl residue,
- R²¹ =: 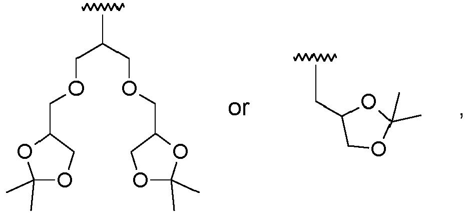
- X =: independently from other X residues in the same molecule: -O or -NH,
- m =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- n =: 10 to 500, in particular 20 to 450, in particular 30 to 400, in particular 40 to 350, in particular 50 to 300, in particular 60 to 250, in particular 70 to 200, in particular 80 to 150, in particular 90 to 100, and
- s =: independently from other s indices in the same molecule: 1 to 12, in particular 2 to 11, in particular 3 to 10, in particular 4 to 9, in particular 5 to 8, in particular 6 to 7.

This step is performed in an organic solvent. Dimethylformamide (DMF) is a particularly appropriate organic solvent. An appropriate reaction time lies in a range of from 6 hours to 24 hours, in particular from 10 hours to 20 hours, in particular from 12 hours to 18 hours, in particular from 14 hours to 16 hours. An appropriate reaction temperature lies in a range of from 20 °C to 100 °C, in particular from 30 °C to 90 °C, in particular from 40 °C to 80 °C, in particular from 50 °C to 75 °C, in particular from 60 °C to 70 °C.

In an optional preceding method step, the compound according to general formula (II) can be synthesized by a reaction between an acryloyl chloride (an prop-2-enoyl chloride) according to general formula (XVII) with a compound according to general formula (XVIII):

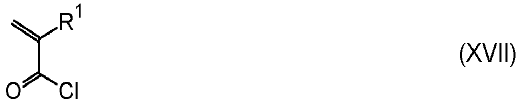

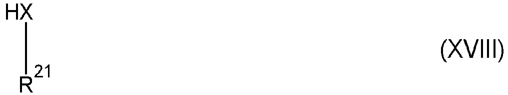

This optional step is performed in an organic solvent. Dichloromethane (DCM) is a particularly appropriate organic solvent. The reaction is furthermore performed in the presence of a base, in particular an organic base. Triethylamine (Et₃N) is a particularly appropriate organic base. An appropriate reaction time lies in a range of from 6 hours to 24 hours, in particular from 10 hours to 20 hours, in particular from 12 hours to 18 hours, in particular from 14 hours to 16 hours. An appropriate reaction temperature lies in a range of from 0 °C to 30 °C, in particular from 5 °C to 25 °C, in particular from 10 °C to 20 °C.

Afterwards, the end groups of residue R²¹ are deprotected to obtain a compound according to general formula (V): 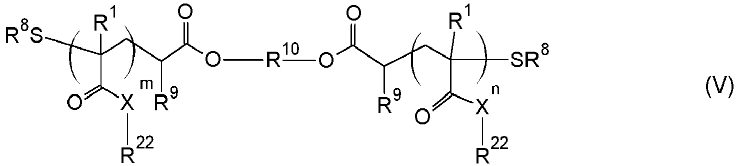 wherein
- R²² =:

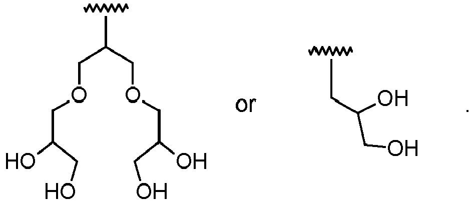

In an embodiment, this deprotection step is done in an acidic aqueous solution of an alcohol. Ethanol is a particularly appropriate alcohol. Hydrochloric acid is a particularly appropriate acid. A concentration of the acid lies in a range of from 1 % to 10 % (w/v), in particular from 2 % to 9 %, in particular from 3 % to 8 %, in particular from 4 % to 7 %, in particular from 5 % to 6 %. An appropriate reaction time lies in a range of from 1 hour to 12 hours, in particular from 3 hours to 10 hours, in particular from 6 hours to 8 hours.

In an optional functionalization step, at least some hydroxyl groups of residue R²² are substituted by at least one of the following residues: -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 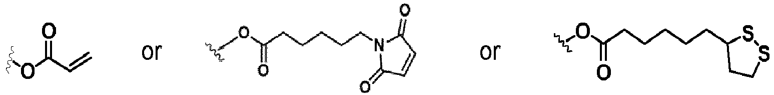 or a salt of any of these residues.

To give an example, a sulfation may be achieved with sulfamic acid (amidosulfonic acid; H₃NSO₃) in an organic solvent. An appropriate organic solvent is DMF, in particular dry DMF. An appropriate reaction time lies in a range of from 12 hours to 48 hours, in particular from 20 hours to 40 hours, in particular from 24 hours to 36 hours. An appropriate reaction temperature lies in a range of from 0 °C to 30 °C, in particular from 5 °C to 25 °C, in particular from 10 °C to 20 °C.

In an embodiment, residue R⁸ corresponds to the following residue in formula (IV):

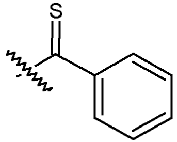

In an optional method step preceding the deprotection step, the compound according to general formula (IV) is reacted with 2,2'-dipyridyldisulfide to obtain a compound according to general formula (VI):

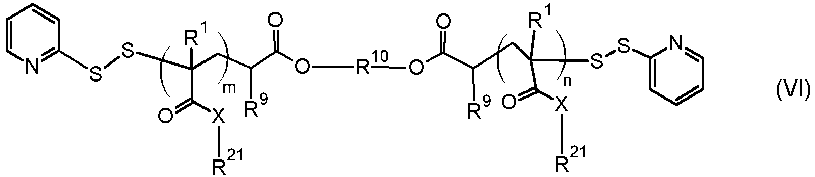

This method step is performed in an organic solvent. Dichloromethane (DCM) is a particularly appropriate organic solvent. In an embodiment, the reaction is furthermore performed in the presence of butylamine. An appropriate reaction time lies in a range of from 1 hour to 12 hours, in particular from 3 hours to 10 hours, in particular from 6 hours to 8 hours. An appropriate reaction temperature lies in a range of from 0 °C to 30 °C, in particular from 5 °C to 25 °C, in particular from 10 °C to 20 °C.

The compound according to general formula (VI) can then be deprotected according to the above explanations to obtain a compound according to general formula (V), wherein residue R⁸ corresponds the following residue:

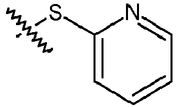

In an embodiment, residue R¹⁰ is -CH₂-CH₂-.

In an embodiment, residue R¹⁰ is one of the following residues:

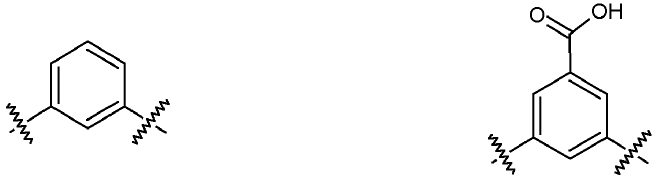

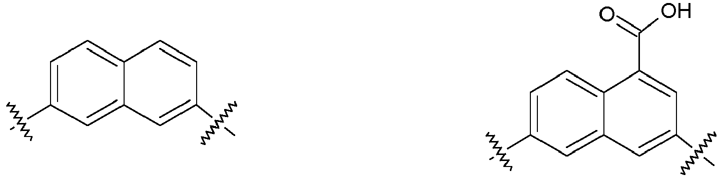

In an aspect, the present invention relates to a second method for manufacturing a polymer according to the preceding explanations. This method comprises the steps explained in the following.

In a method step, a compound according to general formula (II) and a compound according to general formula (VII) are reacted with a compound according to general formula (III) to obtain a compound according to general formula (VIII):

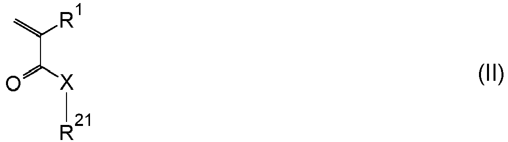

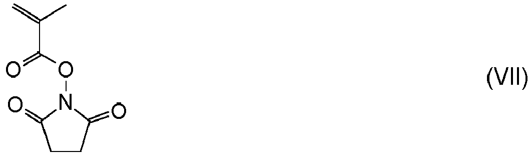

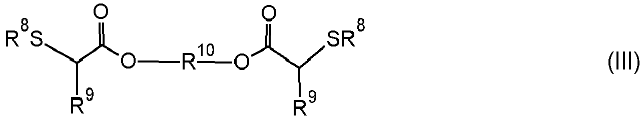

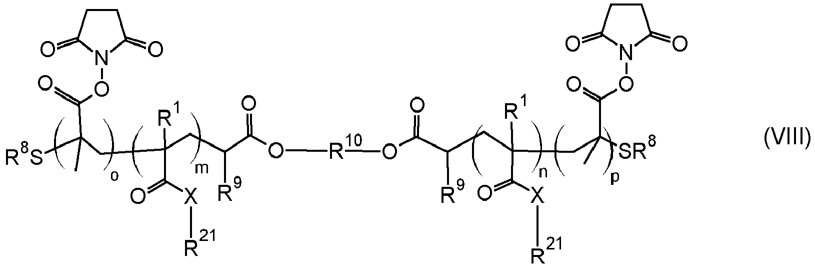

In this context,
- R¹ =: -CH₃ or -H,
- R⁸ =: independently from other R⁸ residues in the same molecule: 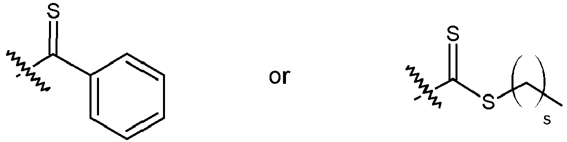 or 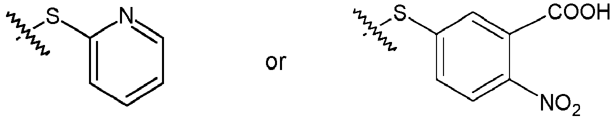 or 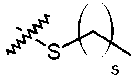
- R⁹ =: independently from other R⁹ residues in the same molecule: a methyl residue or a phenyl residue,
- R¹⁰ =: independently from other R¹⁰ residues in the same molecule: a substituted or non-substituted C₁-C₁₂ alkyl residue or a substituted or non-substituted C₆-C₁₂ aryl residue,
- R²¹ =: 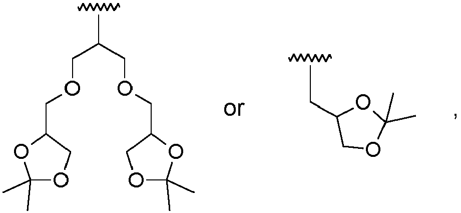
- X =: independently from other X residues in the same molecule: -O or -NH,
- m =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- n =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- o =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11, and
- p =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11.

This step is performed in an organic solvent. DMF is a particularly appropriate organic solvent. An appropriate reaction time lies in a range of from 6 hours to 24 hours, in particular from 10 hours to 20 hours, in particular from 12 hours to 18 hours, in particular from 14 hours to 16 hours. An appropriate reaction temperature lies in a range of from 20 °C to 100 °C, in particular from 30 °C to 90 °C, in particular from 40 °C to 80 °C, in particular from 50 °C to 75 °C, in particular from 60 °C to 70 °C.

In another method step, the compound according to general formula (VIII) is reacted with a compound according to general formula (IX) to obtain a compound according to general formula (X):

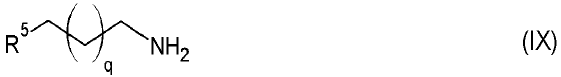

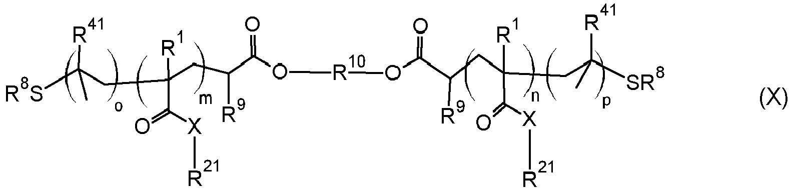

In this context, 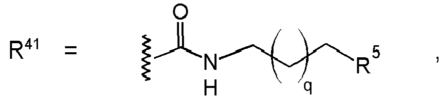 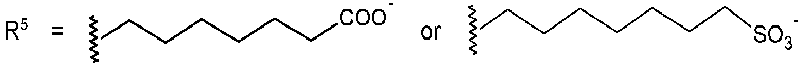 or 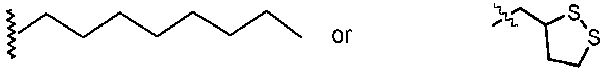 or 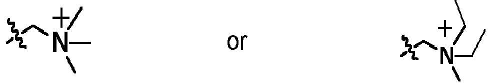 or a salt of any of these residues.

This method step is performed in an organic solvent. DCM is a particularly appropriate organic solvent. An appropriate reaction time lies in a range of from 1 hour to 12 hours, in particular from 3 hours to 10 hours, in particular from 6 hours to 8 hours. An appropriate reaction temperature lies in a range of from 0 °C to 30 °C, in particular from 5 °C to 25 °C, in particular from 10 °C to 20 °C.

Afterwards, the end groups of residue R²¹ are deprotected to obtain a compound according to general formula (XI):

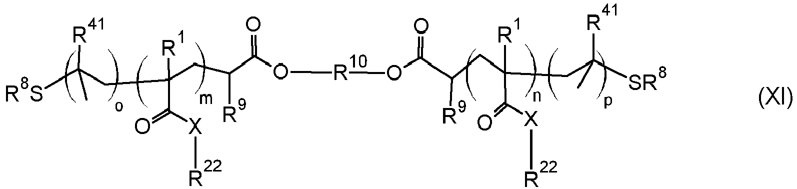

In this context,
- R²² =:

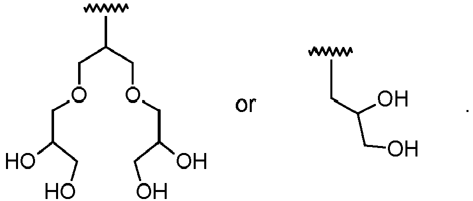

In an embodiment, this deprotection step is done in an acidic aqueous solution of an alcohol. Ethanol is a particularly appropriate alcohol. Hydrochloric acid is a particularly appropriate acid. A concentration of the acid lies in a range of from 1 % to 10 % (w/v), in particular from 2 % to 9 %, in particular from 3 % to 8 %, in particular from 4 % to 7 %, in particular from 5 % to 6 %. An appropriate reaction time lies in a range of from 1 hour to 12 hours, in particular from 3 hours to 10 hours, in particular from 6 hours to 8 hours.

In an optional functionalization step, at least some hydroxyl groups of residue R²² are substituted by at least one of the following residues: -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 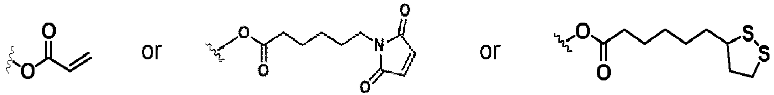 or a salt of any of these residues.

In an aspect, the present invention relates to a third method for manufacturing a polymer according to the preceding explanations. This method can be denoted as statistical copolymer synthesis and comprises the steps explained in the following.

In a method step, a compound according to general formula (II) and a compound according to general formula (XII) are reacted with a compound according to general formula (III) to obtain a compound according to general formula (XIII):

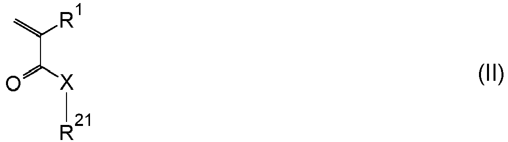

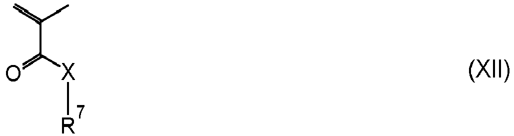

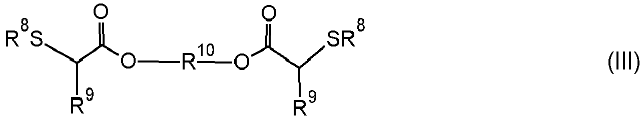

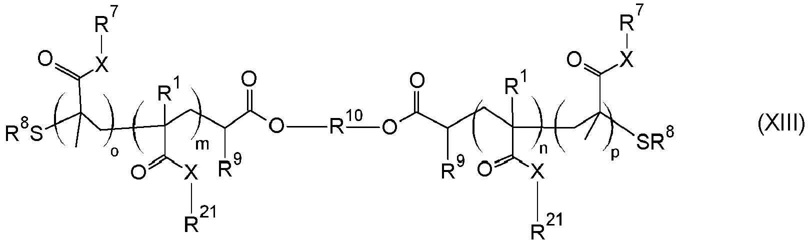

In this context,
- R¹ =: -CH₃ or -H,
- R⁷ =: independently from other R⁷ residues in the same molecule: -(CH₂)ᵣCH₃ or 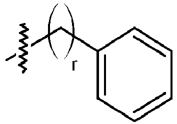 or 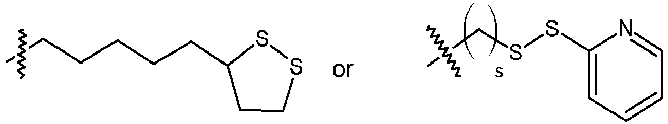 or 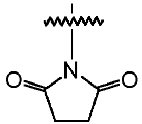
- R⁸ =: independently from other R⁸ residues in the same molecule: 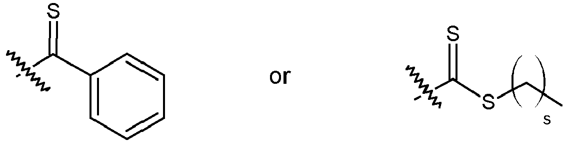 or 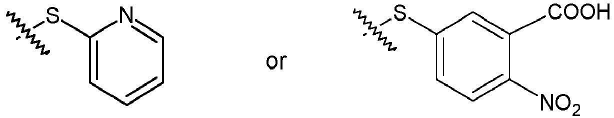 or 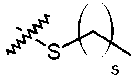
- R⁹ =: independently from other R⁹ residues in the same molecule: a methyl residue or a phenyl residue,
- R¹⁰ =: independently from other R¹⁰ residues in the same molecule: a substituted or non-substituted C₁-C₁₂ alkyl residue or a substituted or non-substituted C₆-C₁₂ aryl residue,
- R²¹ =: 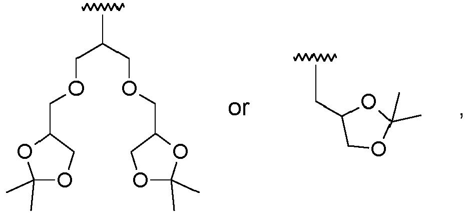
- X =: independently from other X residues in the same molecule: -O or -NH,
- m =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- n =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- o =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- p =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- r =: independently from other r indices in the same molecule: 0 to 11, in particular 1 to 10, in particular 2 to 9, in particular 3 to 8, in particular 4 to 7, in particular 5 to 6, and
- s =: independently from other s indices in the same molecule: 1 to 12, in particular 2 to 11, in particular 3 to 10, in particular 4 to 9, in particular 5 to 8, in particular 6 to 7.

This step is performed in an organic solvent. DMF is a particularly appropriate organic solvent. An appropriate reaction time lies in a range of from 6 hours to 24 hours, in particular from 10 hours to 20 hours, in particular from 12 hours to 18 hours, in particular from 14 hours to 16 hours. An appropriate reaction temperature lies in a range of from 20 °C to 100 °C, in particular from 30 °C to 90 °C, in particular from 40 °C to 80 °C, in particular from 50 °C to 75 °C, in particular from 60 °C to 70 °C.

In a further method step, the end groups of residue R²¹ are deprotected to obtain a compound according to general formula (XIV):

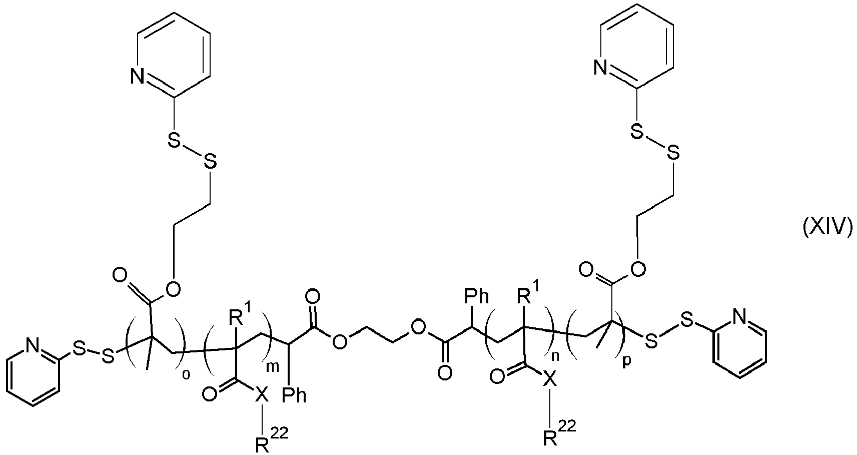

In this context,
- R²² =:

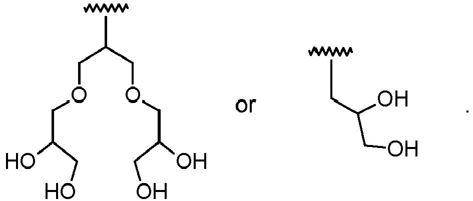

In an embodiment, this deprotection step is done in an acidic aqueous solution of an alcohol. Ethanol is a particularly appropriate alcohol. Hydrochloric acid is a particularly appropriate acid. A concentration of the acid lies in a range of from 1 % to 10 % (w/v), in particular from 2 % to 9 %, in particular from 3 % to 8 %, in particular from 4 % to 7 %, in particular from 5 % to 6 %. An appropriate reaction time lies in a range of from 1 hour to 12 hours, in particular from 3 hours to 10 hours, in particular from 6 hours to 8 hours.

In an optional functionalization step, at least some hydroxyl groups of residue R²² are substituted by at least one of the following residues: -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 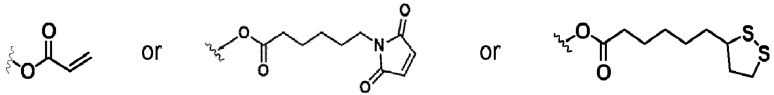 or a salt of any of these residues.

In an embodiment, residue R⁸ corresponds to the following residue in formula (XIII):

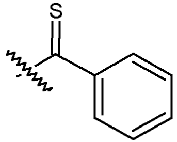

In an optional method step preceding the deprotection step, the compound according to general formula (XIII) is reacted with 2,2'-dipyridyldisulfide to obtain a compound according to general formula (XV):

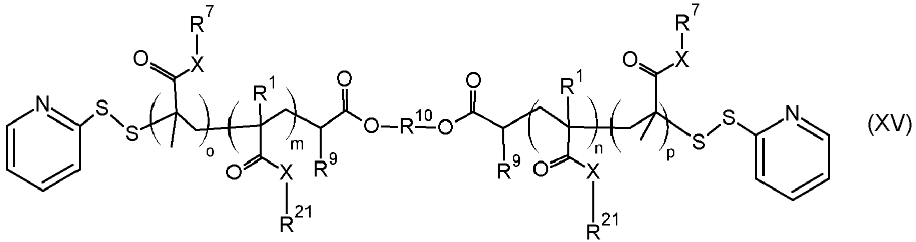

The compound according to general formula (XV) can then be deprotected according to the above explanations to obtain a compound according to general formula (XIV), wherein residue R⁸ corresponds the following residue:

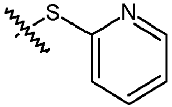

This method step is performed in an organic solvent. DCM is a particularly appropriate organic solvent. In an embodiment, the reaction is furthermore performed in the presence of butylamine. An appropriate reaction time lies in a range of from 1 hour to 12 hours, in particular from 3 hours to 10 hours, in particular from 6 hours to 8 hours. An appropriate reaction temperature lies in a range of from 0 °C to 30 °C, in particular from 5 °C to 25 °C, in particular from 10 °C to 20 °C.

In an aspect, the present invention relates to a fourth method for manufacturing a polymer according to the preceding explanations. This method can be denoted as block copolymer synthesis and comprises the steps explained in the following.

In a method step, a compound according to general formula (IV) is reacted with a compound according to general formula (XII) to obtain a compound according to general formula (XIII):

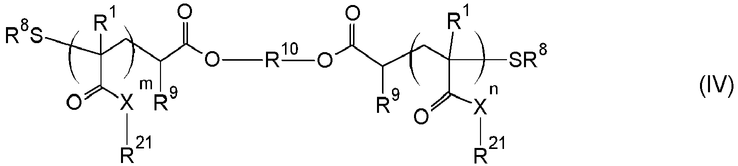

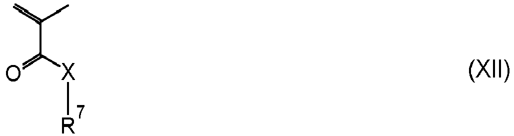

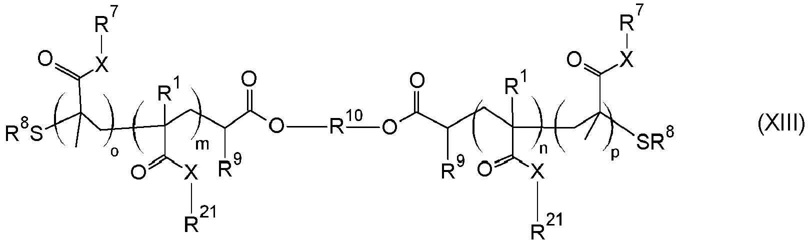

In this context,
- R¹ =: -CH₃ or -H,
- R⁷ =: independently from other R⁷ residues in the same molecule: 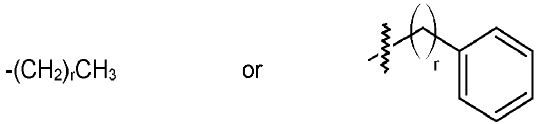 or 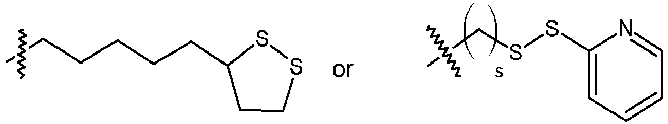 or 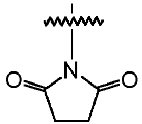
- R⁸ =: independently from other R⁸ residues in the same molecule: 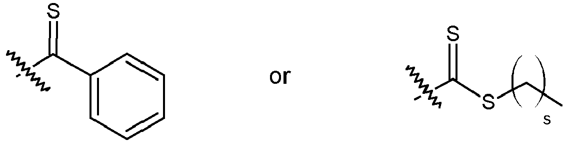 or 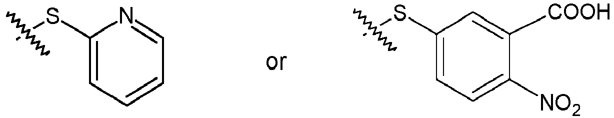 or 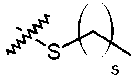
- R⁹ =: independently from other R⁹ residues in the same molecule: a methyl residue or a phenyl residue,
- R¹⁰ =: independently from other R¹⁰ residues in the same molecule: a substituted or non-substituted C₁-C₁₂ alkyl residue or a substituted or non-substituted C₆-C₁₂ aryl residue,
- R²¹ =: 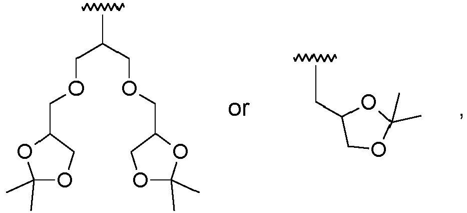
- X =: independently from other X residues in the same molecule: -O or -NH,
- m =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- n =: 10 to 500, in particular 20 to 450, in particular 30 to 400, in particular 40 to 350, in particular 50 to 300, in particular 60 to 250, in particular 70 to 200, in particular 80 to 150, in particular 90 to 100,
- o =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- p =: 0 to 50, in particular 1 to 40, in particular 2 to 30, in particular 3 to 25, in particular 4 to 20, in particular 5 to 15, in particular 6 to 14, in particular 7 to 13, in particular 8 to 12, in particular 9 to 11,
- r =: independently from other r indices in the same molecule: 0 to 11, in particular 1 to 10, in particular 2 to 9, in particular 3 to 8, in particular 4 to 7, in particular 5 to 6, and
- s =: independently from other s indices in the same molecule: 1 to 12, in particular 2 to 11, in particular 3 to 10, in particular 4 to 9, in particular 5 to 8, in particular 6 to 7.

This step is performed in an organic solvent. DMF is a particularly appropriate organic solvent. An appropriate reaction time lies in a range of from 6 hours to 24 hours, in particular from 10 hours to 20 hours, in particular from 12 hours to 18 hours, in particular from 14 hours to 16 hours. An appropriate reaction temperature lies in a range of from 20 °C to 100 °C, in particular from 30 °C to 90 °C, in particular from 40 °C to 80 °C, in particular from 50 °C to 75 °C, in particular from 60 °C to 70 °C.

Afterwards, a deprotection step like in the third manufacturing method and optionally also a functionalization step like in the third manufacturing method are performed. Reference is made to the explanations given above with respect to these method steps.

The resulting polymer is a block copolymer, in which a plurality of building blocks carrying the index o are arranged adjacent to each other, followed by a block containing a plurality of adjacent building blocks carrying the index m, followed by residue R¹⁰ and the chemical groups flanking residue R¹⁰, followed by a block containing a plurality of adjacent building blocks carrying the index n, and this finally followed by a block containing a plurality of adjacent building blocks carrying the index p. Since the building blocks carrying the indices o and p can be designed such that they have a significantly higher hydrophobicity than the building blocks carrying the indices m and n, it is possible to construe a block copolymer according to the scheme A-B-A, in which A refers to hydrophobic blocks located at the periphery of the block copolymer (made up by the building blocks carrying the indices o and p), and B denotes a hydrophilic central portion of the block copolymer (made up by the building blocks carrying the indices m and n).

In an embodiment, a ratio between blocks carrying the indices m and n and blocks carrying the indices o and p lies in a range of from 1:1 to 10:1, in particular from 2:1 to 9:1, in particular from 3:1 to 8:1, in particular from 4:1 to 7:1, in particular from 5:1 to 6:1.

In an embodiment of the block copolymer synthesis, X is -O and residue R⁷ is 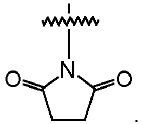 Then, it is possible to manufacture an N-hydroxysuccinimide (NHS) containing block copolymer. It is also possible to further react the NHS group, as explained above with respect to the second manufacturing method.

In an embodiment of any of the precedingly explained manufacturing methods, a functionalization of the polymer (i.e., of at least some of the hydroxyl groups of residue R²² of the resulting compound) with a sugar is carried out. This sugar functionalization is done, in an embodiment, by reacting a compound according to general structure (XI) or (XIV) with methanesulfonyl chloride (mesyl chloride; MsCl), an organic base, in particular triethylamine, and sodium azide (NaN₃). This reaction is done in an organic solvent, wherein DMF is a particular appropriate organic solvent. This reaction leads to a substitution of at least some of the hydroxyl groups of residue R²² by azide residues. The degree of substitution (and thus the degree of functionalization) can be adjusted, e.g., by choosing an appropriate reaction time.

Subsequently, an ethynylated sugar, in particular an ethynylated sialic acid, an ethynylated mannose, an ethynylated fucose, or an ethynylated galactose, is reacted with the azide-functionalized polymer. This results in a sugar functionalization of the polymer by click chemistry.

In an aspect, the present invention relates to a method for manufacturing a hydrogel from any of the precedingly explained polymers. Such hydrogel is formed by allowing a reaction between any of the above-explained polymers with a crosslinker chosen from the group consisting of a PEG-dithiol, a 4-arm PEG-tetrathiol, and a PEG-dicyclooctyne corresponding to the following formula:

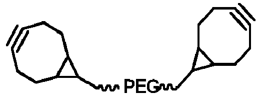

Alternatively, ultraviolet light may be used as cross-linking initiator. The formed hydrogel comprises a porous network with a heterogeneous pore size lying in a range of from 500 nm to several micrometers. Since the hydrogel formation is, amongst others, due to disulfide linkages between individual polymer molecules and between polymer molecules and crosslinker molecules, the hydrogel can be degraded by the addition of glutathione (GSH). Depending on the GSH concentration, a complete degradation of the polymer-based hydrogel is possible. To give an example, in the presence of 10 mM GSH, a complete degradation of the hydrogel occurs within 72 hours.

All embodiments of the polymer can be combined in any desired way and can be transferred either individually or in any arbitrary combination to the various uses, to the hydrogel, to the pharmaceutical composition, and to the different manufacturing methods. Likewise, all embodiments of the uses can be combined in any desired way and can be transferred either individually or in any arbitrary combination to the polymer, to the hydrogel, to the pharmaceutical composition, and to the different manufacturing methods. Furthermore, all embodiments of the hydrogel can be combined in any desired way and can be transferred either individually or in any arbitrary combination to the polymer, to the various uses, to the pharmaceutical composition, and to the different manufacturing methods. All embodiments of the pharmaceutical composition can be combined in any desired way and can be transferred either individually or in any arbitrary combination to the polymer, to the various uses, to the hydrogel, and to the different manufacturing methods. Finally, all embodiments of the different manufacturing methods can be combined in any desired way and can be transferred either individually or in any arbitrary combination to the polymer, to the various uses, to the hydrogel, to the pharmaceutical composition, and to the respective other manufacturing method.

Further details of aspects of the present invention will be explained in the following making reference to exemplary embodiments and accompanying Figures. In the Figures:
- Figure 1: shows the structure of polymers P1, P1a, P1b, P1c, P2, and P3;
- Figures 2A to 2C: show the dose dependent inhibition capability of different polymers against a SARS-CoV-2 infection of cells;
- Figures 3A to 3D: show the results of cytotoxicity measurements of different polymers with respect to different cell types;
- Figure 4: shows the anticoagulant activity of different polymers;
- Figures 5A and 5B: show the inhibition capability of different polymers against a HSV-1 infection of cells;
- Figure 6: show the results of cytotoxicity measurements of polymer P22 with respect to different cell types;
- Figure 7: shows the anticoagulant activity of polymer P22;
- Figure 8: shows the rheological properties of a first P22-based hydrogel;
- Figure 9: shows the rheological properties of a second P22-based hydrogel;
- Figure 10: shows the rheological properties of a third P22-based hydrogel; and
- Figure 11: shows a plot illustrating the degradability of a P 22-based hydrogel.

To synthesize different polymers having a structure according to general formula (I), a synthesized bifunctional chain transfer agent (CTA; corresponding to general formula (III)) was reacted with a monomer M1 (OGMA) corresponding to general formula (II) to produce different molecular weight polymers (POGMA) in gram scale. The terminal dithiocarbonate groups of **POGMA** were converted to 2-pyridyl disulfide (PDS) and UV/Vis analysis confirmed the transformation. Quantification [27] of PDS groups indicated that they are at the both chain terminals confirming polymerization grown from the both initiating sites. PDS groups at terminals could help **POGMA** for further polymerization in presence of di-thiol via thiol-disulfide exchange reaction. [28]

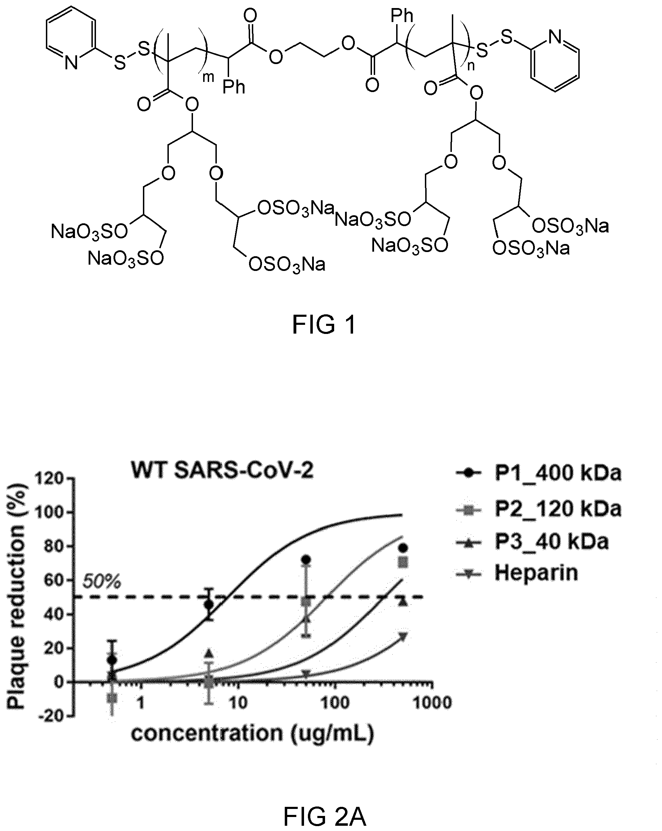

Afterwards, acetonide groups were deprotected to generate hydroxyl functionalities in residue R²¹. By decoration of sulfates groups [29] at the side chain, the final polymers **(P1, P2** and **P3)** were obtained (cf. Figure 1; **P1:** m + n = 580; **P2:** m + n = 180; **P3:** m + n = 60). The abbreviation "Ph" denotes a phenyl residue. The degree of sulfation was quantified by elemental analysis. For simplification reasons only, all hydroxyl groups of the molecule shown in Figure 1 are depicted in their sulfated state. The amount of substituted hydroxyl groups in the real polymers depends on the degree of functionalization.

With this synthetic route, the inventors have successfully synthesized polymer (P1) with highest molecular weight of ~ 430 kDa. It will be referred to in the following also as PGMAS_400 kDa or P1_400 kDa. Likewise, polymer P2 will be referred to in the following also as PGMAS_120 kDa, PGMAS_100 kDa, P2_120 kDa or P2_100 kDa, and polymer P3 as PGMAS_40 kDa or P3_40 kDa.

**Table 9: Characterization and half-maximal inhibitory concentration (IC₅₀) of polymers against SARS-CoV-2 (in µg/mL and in µM).**

| **Polymer** | **MW^{a}(kDa)** | **DOF** | **ζ potential (mV)** | **IC₅₀ (µg/mL)** | **ICso (µM)** |
|---|---|---|---|---|---|
| **P1** | 430 | 94 | -65.1± 1.7 | 2.26 ± 0.82 | 0.0056 ± 0.002 |
| **P2** | 120 | 90 | -48.3 ± 1.3 | 39.5 ±18.2 | 0.329 ± 0.15 |
| **P3** | 40 | 92 | -33.7 ± 1.9 | 209.9 ± 170.5 | 5.24 ± 4.26 |
| **P1a** | 370 | 80 | -55.8± 0.9 | 6.7 ± 0.57 | 0.018 ± 0.0015 |
| **P1b** | 300 | 60 | -31.7± 1.1 | 48.9 ± 44.2 | 0.163 ± 0.147 |
| **P1c** | 205 | 10 | -13.7± 1.6 | 2404 ± 1869 | 11.72 ± 9.12 |
| **P1d** | 436 | 92 | -57.2± 0.7 | 37.01 ± 17.59 | 0.086 ± 0.04 |
| **Heparin** | 13-15 | NA | -31.2± 3.9 | 1383.0 ± 255.1 | 92.3 ± 19.4 |

| | | | | | |
|---|---|---|---|---|---|
| ^{a 1}H NMR determined molecular weight; DOF stands for degree of functionalization (either sulfates or carboxylate); Heparin was used as control. | | | | | |

Overall negative charge of the polymers owing to sulfate groups was confirmed by very high negative value of zeta potential (cf. Table 9). To check the morphology of P1 in aqueous solution, cryo-electron microscopy (cryo-EM) was performed. In accordance with the inventors' hypothesis, worm-like defined fibers having a length between approximately 150 nm and 215 nm were observed. Tomography of the sample was measured to calculate accurate length and width of the fibers. Tomography analysis estimated (selecting 10 random fibers) a length of the fibers lying in a range of from ~ 155-211 nm and their width was ~ 2 nm matching with the theoretical average length ~ 180 nm and width ~ 2 nm of P1 (average repeat unit of P1 = 600) calculated based on the atomic distance in the polymer chain. This indicates that P1 forms single chain fibers [30] in aqueous solution. The observed size distribution of fibers can be explained by considering polydispersity (Ð = 1.5) in sample P1.

To validate whether the polymeric structural construct might be the key parameter for single chain fiber morphology, the inventors have checked cryo-EM morphology of P3, which is 10 times shorter in length than P1. If the hypothesis of single chain fiber with this structural construct was correct, one would expect to get single chain fibers with shortest synthesized polymer P3 as well. Interestingly, it was found that an aqueous solution of P3 showed a fiber structure with a length of ~ 18 nm matching with its theoretical extended polymer chain length. It was even more interesting that owing to the same repeat unit for polymer P1 and P3, the fibers from them showed comparable width (~ 2 nm) further confirming single chain polymer fibers. Therefore, it was concluded that the methacrylate backbone introduces a rigidity in the polymer chain that is further supported by the electrostatic repulsion among the sulfated groups. This enhances the possibilities for further chain extension to attain a mucin-like fiber structure.

To evaluate SARS-CoV-2 inhibition activities of synthetic fibers, the inventors investigated the fibers on wild-type (WT) SARS-CoV-2 variant and determined their activities by plaque reduction assay for infection against Vero-E6 and other cell lines. The morphologies of the cells were recorded after incubation with SARS-CoV-2 in presence or absence of polymer fibers. It was very clear from the results that in presence of P1 most of the cells remained healthy, whereas without polymers the cells were infected by the viruses. The negligible number of infected cells in presence of P1 confirmed its SARS-CoV-2 inhibition activity.

The analysis of dose-dependent virus inhibition curves (Figure 2A) suggested that activity of the synthetic fibers was dependent on the molecular weight, more precisely on the length of the polymer fibers (cf. Table 9). The natural polysulfate heparin was used as control. P1 showed remarkably low half-maximal inhibition concentration (C = 2.26 ±0.82 µg/mL, 5.6 nM) in the nanomolar range (cf. Table 9) which is so far the best reported IC₅₀ value for sulfated inhibitors targeting the receptor binding domain (RBD) of SARS-CoV-2 via electrostatic interaction. Moreover, the IC₅₀ values in Table 9 indicate that P1 is -100 times more active than P3, even though P1 is only 10 times longer than P3. Hence, SARS-CoV-2 inhibition activity appears to be not linearly proportional to the length of fibers. This is further supported when comparing the inhibition activity of P1 to the inhibition activity of P2 (the inhibition activity of P1 is ~ 20 times higher than that of P2, whereas its length is only 3 times bigger than that of P2), indicating that some other parameters may also play a role for inhibition in addition to the length of fibers.

This anomaly could be explained by considering the fact that long chain polymer fibers not only interact with the RBD of SARS-CoV-2 via polyvalent interaction but also provide a steric shielding [31] that could prevent SARS-CoV-2 to bind to the active receptor ACE-2 for infection. On the other hand, owing to their relatively shorter fibers, P2 and P3 interact with the RBD via a less effective polyvalent fashion and probably could not provide enough steric shielding to prevent viral attachment to ACE-2. Therefore, the combined effect might be the reason for remarkable low IC₅₀ value for SARS-CoV-2 inhibition of P1.

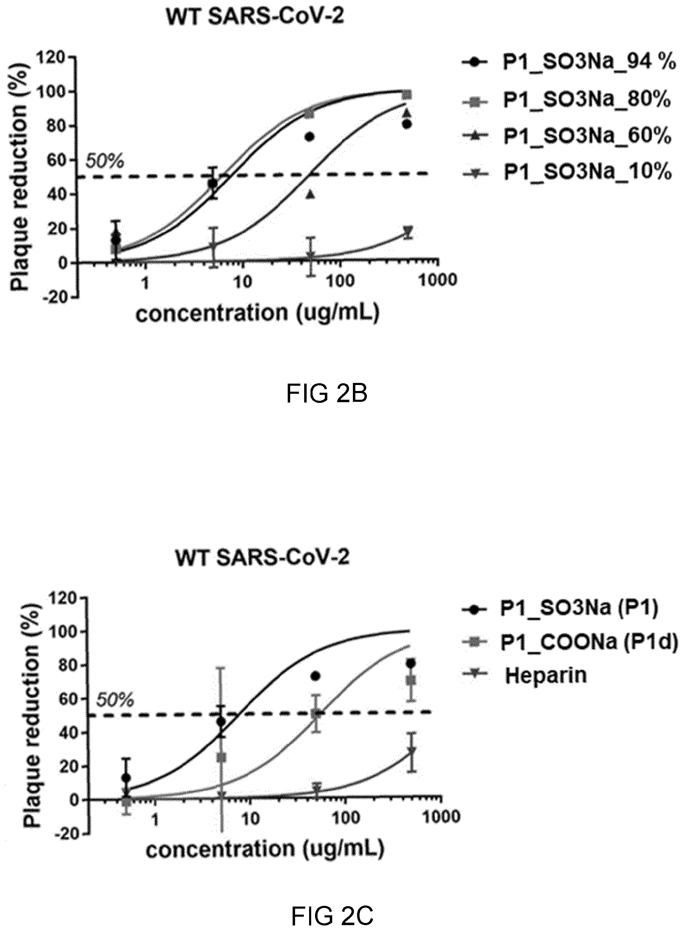

To understand the impact of negatively charged polymers on SARS-CoV-2 inhibition [32], the inventors synthesized derivatives of P1, the most potent inhibitor from the initial studies, by varying the degree of sulfation **(P1a**, **P1b** and **P1c)** and quantified them by elemental analysis. The inventors found that a -10 % sulfated polymer (P1c) showed negligible SARS-CoV-2 inhibition activity (Figure 2B) (IC₅₀ = 2404 ± 1869 µg/mL). With increasing degree of sulfation, the zeta potential becomes more negative (Table 9). At the same time, SARS-CoV-2 inhibition activity increased (Figure 2B). This further confirmed that electrostatic interaction with the RBD of SARS-CoV-2 is responsible for the infection inhibition upon first entry of SARS-CoV-2 into a host cell.

The inventors furthermore synthesized a carboxylated derivative **(P1d)** of polymer **P1.** A plaque reduction assay of **P1d** showed excellent inhibition with IC₅₀ values -37.1 µg/mL (Figure 2C). This confirmed that carboxylate functional groups are also active in SARS-CoV-2 inhibition. However, the sulfated version of **P1** appeared to be more active than its carboxylated version (**P1d**). This could be due to the more electronegative character of the sulfated groups in comparison to the same number of carboxylate groups as observed in zeta potential measurement.

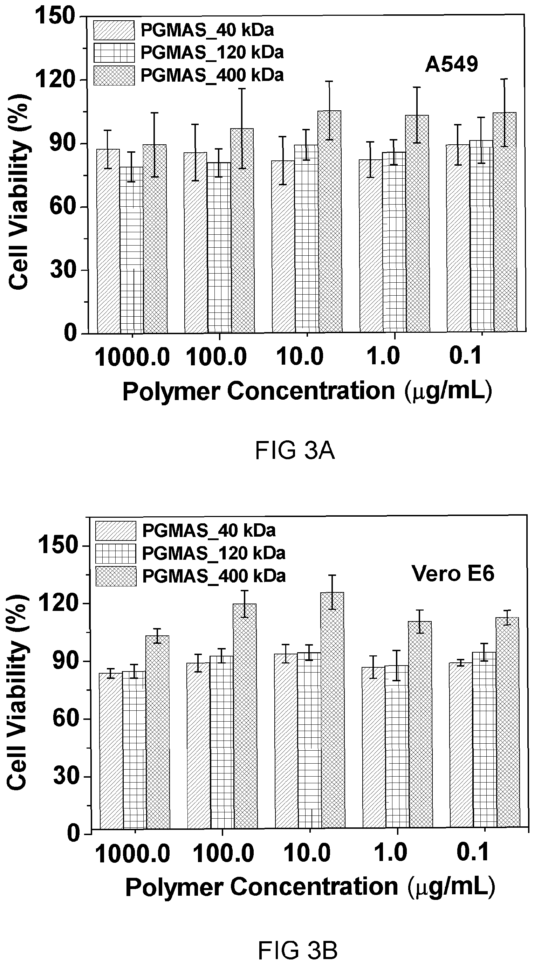

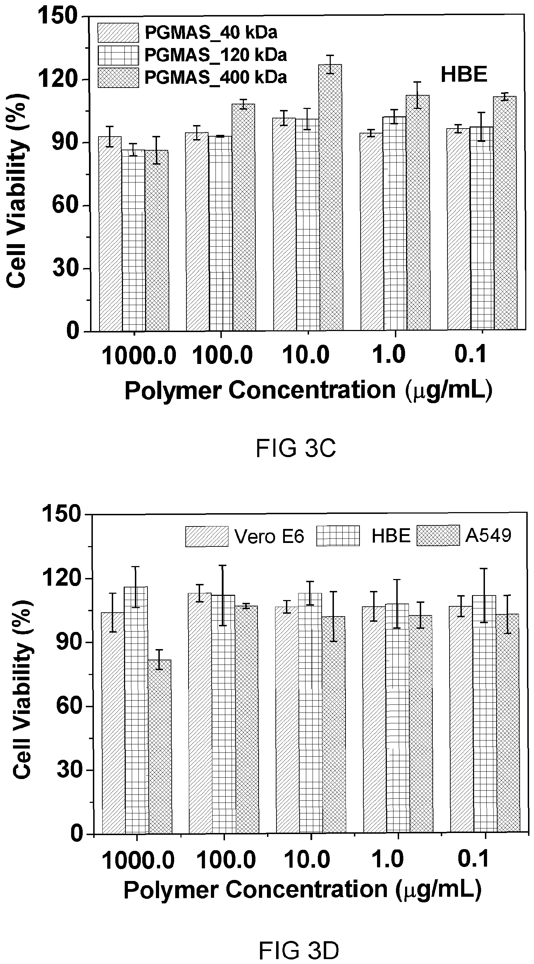

The synthesized polymers, regardless of being functionalized with sulfate groups or carboxylate groups, appeared as highly cell viable in different cell lines (Vero-E6, 16HBE14o and A549) (Figures 3A to 3C for the sulfated polymers and Figure 3D for the carboxylated polymer).

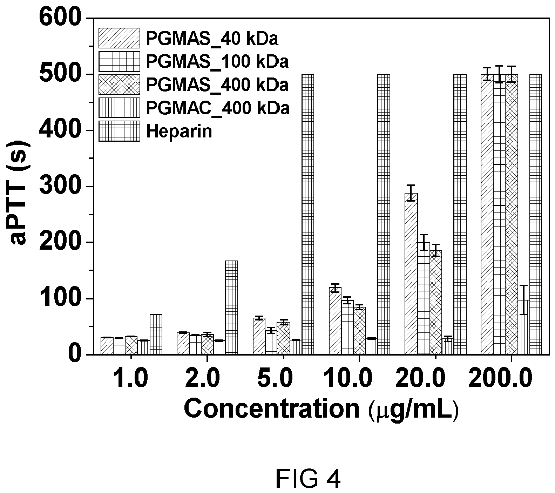

The inherent anticoagulant activity restricts many sulfated compounds from direct applications. In this context, the synthesized functionalized polymers showed negligible anticoagulant activity in comparison to heparin (Figure 4). The anticoagulant activity increased with decreasing length of polymer chain [35]. The carboxylated version of P1 appeared as less anticoagulant than its sulfated version.

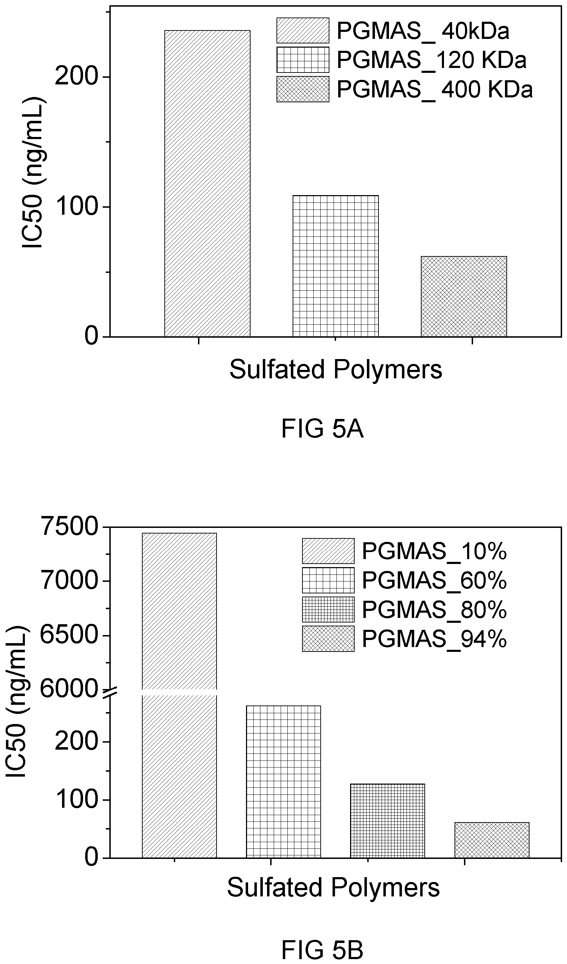

Polymers **P1, P1a, P1b, P1c, P2** and **P3** were also tested on other sulfate binding respiratory viruses, for example Herpes simplex virus-1 (HSV-1), and also showed here their high inhibition activity [34] (Figures 5A and 5B) in comparison to the negative control heparin. The experimental results are summarized in Table 10. Hence, the synthetic polymers appeared as broad-spectrum novel respiratory virus inhibitors.

**Table 10: Inhibition activity of different polymers against HSV-1.**

| **Polymer** | **Figure** | **IC₅₀ (ng/mL)** | **IC₅₀ (nM)** |
|---|---|---|---|
| **PGMAS_40 kDa (P3)** | 5A | 235.7 | 5.89 |
| **PGMAS_120 kDa (P2)** | 5A | 108.8 | 0.91 |
| **PGMAS_400 kDa (P1)** | 5A and 5B | 62.1 | 0.15 |
| **PGMAS_10% (P1c)** | 5B | 7447 | 32.8 |
| **PGMAS_60% (P1b)** | 5B | 262.1 | 0.873 |
| **PGMAS_80% (P1a)** | 5B | 127.8 | 0.345 |

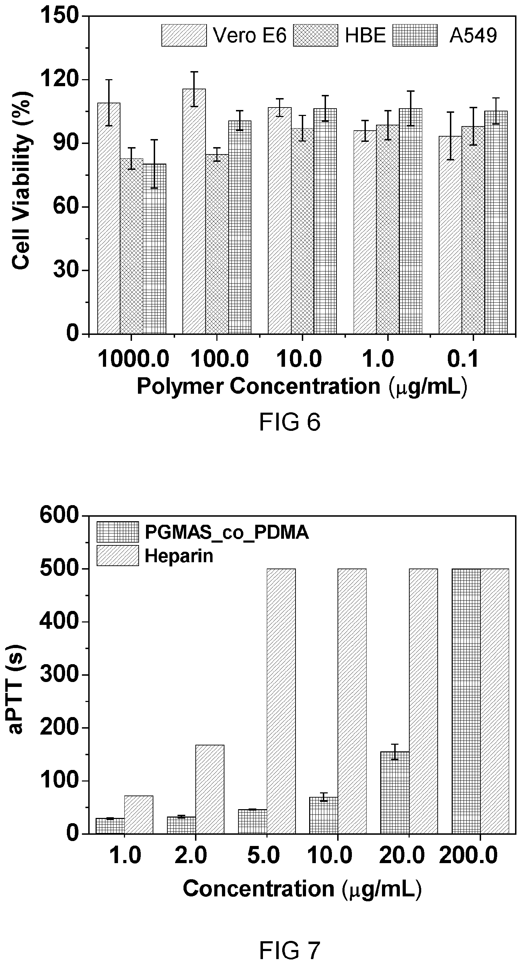

Polymer P22 (cf. Table 5) turned out to be particularly appropriate for hydrogel formation. This polymer has a molecular weight of approximately 200 kDa, a degree of polymerization of 300 and a block ratio between o and p blocks on the one hand and m and n blocks on the other hand (cf. general formula (I)) of approximately 1:9. Due to its suitability for hydrogel formation, P22's SARS-CoV-2 inhibition capacity, as well as its biocompatibility with respect to cytotoxicity and coagulation properties was also tested. P22 turned out to have an IC₅₀ value of SARS-CoV-2 inhibition of 3.5 µg/ml. The results of the experiments with respect to cytotoxicity of P22 are depicted in Figure 6, and the results of the experiments with respect to the coagulation properties of P22 are depicted in Figure 7. Obviously, P22 shows only a very low cytotoxicity and has low anticoagulant properties, at least in concentrations of less than 200 µg/ml.

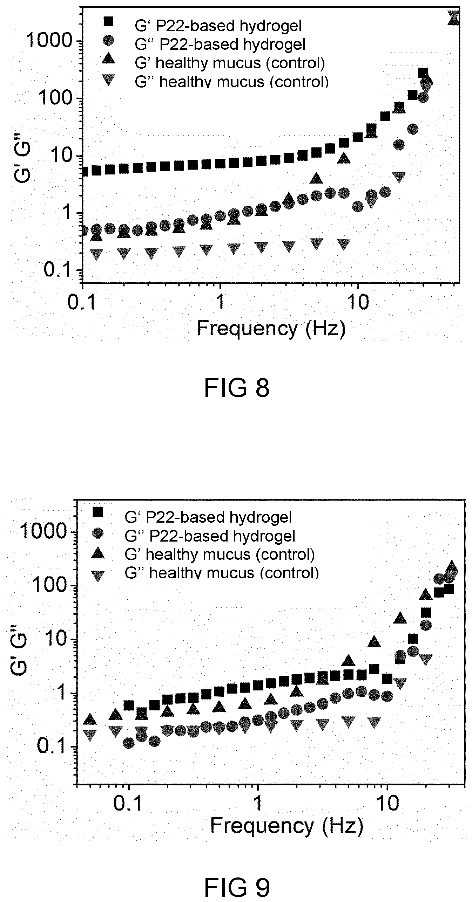

Polymer P22 was then used to form a hydrogel together with HS-PEG-SH (having a molecular weight of approximately 5.0 kDa). The polymer concentration was kept constant at approximately 2.0 weight percent (wt %) at different PEG:polymer ratios. The storage modulus and the loss modulus at 1.0 Hz were determined. The results are depicted in Figure 8 and summarized in the following Table 11. For better comparison, Figure 8 also illustrates the storage modulus and the loss modulus of mucus of a healthy individual.

**Table 11: Composition and physical properties of different hydrogels made from polymer P22 and HS-PEG-SH.**

| **PEG** : **Polymer** | **Polymer / wt %** | **PEG / wt %** | **Storage Modulus (G')** | **Loss Modulus (G")** |
|---|---|---|---|---|
| 1:1 | 2 | 1 | 7.3 | 0.68 |
| 2:1 | 2 | 2 | 7.0 | 0.88 |
| 1:2 | 2 | 0.5 | 0.51 | 0.05 |

Under this experimental setup, a concentration of 2 wt % polymer is very well suited to produce mucus mimetic hydrogels.

Polymer P22 was furthermore used to form a hydrogel together with a 4-arm PEG-tetrathiol corresponding to the following general formula (XIX) (having a molecular weight of approximately 5.0 kDa), wherein n lies in a range of from 10 to 100, in particular 20 to 90, in particular 30 to 80, in particular 40 to 70, in particular 50 to 60:

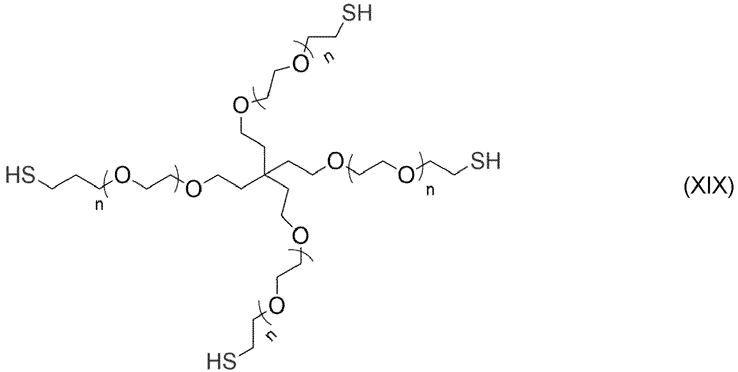

The polymer concentration was varied between 2 and 4 weight percent (wt %) at a constant PEG:polymer ratio of 1:1. The storage modulus and the loss modulus at 1.0 Hz were determined. The results are depicted in Figure 9 and summarized in the following Table 12. For better comparison, Figure 9 also illustrates the storage modulus and the loss modulus of mucus of a healthy individual.

**Table 12: Composition and physical properties of different hydrogels made from polymer P22 and a 4-arm PEG-tetrathiol corresponding to the following general formula (XIX).**

| **PEG** : **Polymer** | **Polymer / wt %** | **PEG / wt %** | **Storage Modulus (G')** | **Loss Modulus (G")** |
|---|---|---|---|---|
| 1:1 | 2 | 0.4 | 2.1 | 0.9 |
| 1:1 | 3 | 0.6 | 35.3 | 1.9 |
| 1:1 | 4 | 0.8 | 270 | 13.2 |

Under this experimental setup, a concentration of 2 to 3 wt % polymer is very well suited to produce mucus mimetic hydrogels.

Polymer P22 was furthermore used to form a hydrogel together with a 4-arm PEG-tetrathiol corresponding to general formula (XIX) (having a molecular weight of approximately 10.0 kDa), wherein n lies in a range of from 10 to 100, in particular 20 to 90, in particular 30 to 80, in particular 40 to 70, in particular 50 to 60.

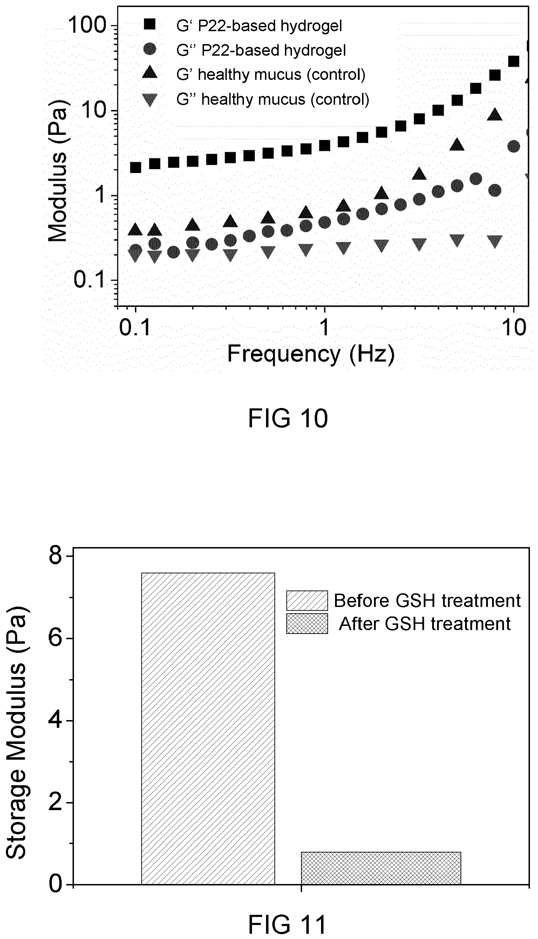

The polymer concentration was kept at 2 weight percent (wt %) at a constant PEG:polymer ratio of 1:1. Due to the higher molecular weight of the crosslinker in comparison to the previously explained embodiment, the amount of PEG was twice as high as in the previously explained embodiment to achieve the same PEG:polymer ratio. The storage modulus and the loss modulus at 1.0 Hz were determined. The results are depicted in Figure 10 and summarized in the following Table 13. For better comparison, Figure 10 also illustrates the storage modulus and the loss modulus of mucus of a healthy individual.

**Table 13: Composition and physical properties of different hydrogels made from polymer P22 and a 4-arm PEG-tetrathiol corresponding to the following general formula (XIX).**

| **PEG** : **Polymer** | **Polymer / wt %** | **PEG / wt %** | **Storage Modulus (G')** | **Loss Modulus (G")** |
|---|---|---|---|---|
| 1:1 | 2 | 0.8 | 3.86 | 0.9 |

Under this experimental setup, a concentration of 1.5 to 2 wt % polymer is very well suited to produce mucus mimetic hydrogels.

In addition, the hydrogel degradability was tested. The structure of the hydrogels is determined to a relevant extent by disulfide bridges. The storage modulus of the formed hydrogels was tested as explained above. Afterwards, glutathione was added in a concentration of 10 mM to the hydrogels. After 72 hours, the modulus was tested again under identical conditions. As can be seen from Figure 11, the modulus significantly decreased after the glutathione treatment, indicating that the hydrogels were fully degraded by glutathione due to breakup of the disulfide bridges. This clearly indicates that the hydrogels are easily degradable. In this context, the degradation is dependent on glutathione concentration.

In summary, a methodology for gram-scale synthesis of methacrylate-based functionalized dendronized polyglycerol polymers was developed. These polymers appeared as highly biocompatible and less anticoagulant, formed thread-like single chain fibers and showed excellent inhibition against respiratory viruses such as SARS-CoV-2 and HSV-1. They could be easily used for forming degradable hydrogels together with a crosslinker.

### List of references cited in the preceding sections or deemed otherwise relevant

[1] Zanin, M.; Baviskar, P.; Webster, R.; Webby, R. Cell Host Microbe. 2016, 19, 159-168.
[2] Werlang, C.; Cárcarmo-Oyarce, G.; Ribbeck, K. Nat. Rev. Mater. 2019, 4, 134-145.
[3] Ridley, C.; Thornton, D. J. Biochem. Soc. Trans. 2018, 46, 1099-1106.
[4] Wheeler, K. M.; Cárcamo-Oyarce, G.; Turner, B. S.; Dellos-Nolan, S.; Co1, J. Y.; Lehoux, S.; Cummings, R. D.; Wozniak, D J.; Ribbeck, K. Nat. Microbiol. 2019, 4, 2146-2154.
[5] Lieleg, O.; Lieleg, C.; Bloom, J.; Buck, C. B.; Ribbeck, K. Biomacromolecules 2012, 13, 1724-1732.
[6] Wardzala, C. L.; Wood, A. M.; Belnap, D. M.; Kramer, J. R. ACS Cent. Sci. 2022, 8, 351-360.
[7] Song, D.; Iverson, E.; Kaler, L.; Bader, S.; Scull, M. A.; Duncan, G. A. ACS Biomater. Sci. Eng. 2021, 7, 2723-2733.
[8] Thornton, D. J.; Sheehan, J. K. Proc. Am. Thorac. Soc. 2004, 1, 54-61.
[9] Authimoolam, S. P.; Dziubla, T. D. Polymers 2016, 8, 71.
[10] Javitt, G.; Khmelnitsky, L.; Albert, L.; Bigman, L. S.; Elad, N.; Morgenstern, D.; Ilani, T.; Levy, Y.; Diskin, R.; Fass, D. Cell 2020, 183, 717-729.
[11] Rabuka, D.; Parthasarathy, R.; Lee, G. S.; Chen, X.; Groves, J. T.; Bertozzi, C. R. J. Am. Chem. Soc. 2007, 129, 5462-5471.
[12] Kruger, A. G.; Brucks, S. D.; Yan, T.; Cárcarmo-Oyarce, G.; Wei, Y.; Wen, D. H.; Carvalho, D. R.; Hore, M. J. A.; Ribbeck, K.; Schrock, R. R.; Kiessling, L. L. ACS Cent. Sci. 2021, 7, 624-630.
[13] Kwan, C.-S.; Cerullo, A. R.; Braunschweig, A. B. ChemPlusChem 2020, 85, 2704-2721.
[14] Kramer, J. R.; Onoa, B.; Bustamante, C.; Bertozzi, C. R. Proc. Natl. Acad. Sci. U. S. A. 2015, 112, 12574-12579.
[15] Cohen, M., Senaati, H. P.; Fisher, C. J.; Huang, M. L.; Gagneux, P.; Godula, K. ACS Cent. Sci. 2016, 2, 710-714.
[16] Wallert, M.; Nie, C.; Anilkumar, P.; Abbina, S.; Bhatia, S.; Ludwig, K.; Kizhakkedathu, J. N.; Haag, R.; Block, S. Small, 2020, 16, 2004635
[17] Bej, R.; Haag, R. J. Am. Chem. Soc, 2022, XXXX
[18] M. Calderon, M. A. Quadir, S. K. Sharma, R. Haag, Adv. Mater. 2010, 22, 190-218.
[19] Ye, T.; Zhong, Z.; Garcia-Sastre, A.; Schotsaert, M. Angew. Chem. Int. Ed. 2020, 59, 18885-18897.
[20] T. Shapira, I. A. Monreal, S. P. Dion, D. W. Buchholz, B. Imbiakha, A. D. Olmstead, M. Jager, A. Désilets, G. Gao, M. Martins, T. Vandal, C. A. H. Thompson, A. Chin, W. D. Rees, T. Steiner, I. R. Nabi, E. Marsault, J. Sahler, D. G. Diel, G. R. Van de Walle, A. August, G. R. Whittaker, P.-L. Boudreault, R. Leduc, H. C. Aguilar, F. Jean, Nature, 2022,
[21] A. Luttens, H. Gullberg, E. Abdurakhmanov, D. D. Vo, D. Akaberi, V. O. Talibov, N. Nekhotiaeva, L. Vangeel, S. De Jonghe, D. Jochmans, J. Krambrich, A. Tas, B. Lundgren, Y. Gravenfors, A. J. Craig, Y. Atilaw, A. Sandström, L. W. K. Moodie, Å. Lundkvist, M. J. van Hemert, J. Neyts, J. Lennerstrand, J. Kihlberg, K. Sandberg, U. H. Danielson, J. Carlsson, J. Am. Chem. Soc. 2022, 144,7,2905-2920.
[22] Larue, R. C.; Xing, E.; Kenney, A. D.; Zhang, Y.; Tuazon, J. A.; Li, J.; Yount, J. S.; Li, P.-K. Sharma, A. Bioconjugate Chem. 2021, 32, 215-223.
[23] T. M. Clausen, D. R. Sandoval, C. B. Spliid, J. Pihl, H. R. Perrett, C. D. Painter, A. Narayanan, S. A. Majowicz, E. M. Kwong, R. N. McVicar, B. E. Thacker, C. A. Glass, Z. Yang, J. L. Torres, G. J. Golden, P. L. Bartels, R. N. Porell, A. F. Garretson, L. Laubach, J. Feldman, X. Yin, Y. Pu, B. M. Hauser, T. M. Caradonna, B. P. Kellman, C. Martino, P. L.S.M. Gordts, S. K. Chanda, A. G. Schmidt, K. Godula, S. L. Leibel, J. Jose, K. D. Corbett, A. B. Ward, A. F. Carlin, J. D. Esko, Cell 183, 1043-1057.
[24] Nie, C.; Pouyan, P.; Lauster, D.; Trimpert, J.; Kerkhoff, Y.; Szekeres, G. P.; Wallert, M.; Block, S.; Sahoo, A. K.; Dernedde et al. Angew. Chem. Int. Ed. 2021, 60, 15870-15878.
[25] Donskyi, I. S.; Nie, C.; Kai Ludwig, Jakob Trimpert, Rameez Ahmed, Elisa Quaas, Katharina Achazi, Jörg Radnik, Mohsen Adeli, Rainer Haag, and Klaus Osterrieder, Small 2021, 17, 2007091.
[26] E. Mohammadifar, V. Ahmadi, M. Fardin Gholami, A. Oehrl, O. Kolyvushko, C. Nie, I. S. Donskyi, S. Herziger, J. Radnik, K. Ludwig, C. Böttcher, J. P. Rabe, K. Osterrieder, W. Azab, R, Haag, M. Adeli, Adv. Funct. Mater. 2021, 31, 2009003.
[27] R. Bej, P. Rajdev, R. Barman, S. Ghosh, Polym. Chem. 2020, 11, 990.
[28] R. Bej, S. Ghosh, Eds. Zhang, X.-H., Theato, P. (Wiley-VCH), 2021, ISBN: 978-3-527-34670-7.
[29] F. Paulus, D. Steinhilber, P. Welker, D. Mangoldt, K. Licha, H. Depner, S. Sigrist, R. Haag, Polym. Chem. 2014, 5, 5020.
[30] Y. Yamauchi, N, N. Horimoto, K. Yamada, Y. Matsushita, M. Takeuchi, Y. Ishida, Angew. Chem. Int. Ed. 2021, 60, 1528 -1534.
[31] J. Vonnemann, S. Liese, C. Kuehne, K. Ludwig, J. Dernedde, C. Böttcher, R. R. Netz, R. Haag, J. Am. Chem. Soc. 2015, 137, 2572-2579.
[32] C. Nie, A. K. Sahoo, R. R. Netz, A. Hermann, M. Ballauff, R. Haag, ChemBioChem, 2022, 23, e2021006.
[33] Leal, J.; Smyth, H. D. C.; Ghosh, D. Int. J. Pharm. 2017, 532, 555-572.
[34] Pouyan, P.; Nie, C.; Bhatia, S.; Wedepohl, S.; Achazi, K.; Osterrieder, N.; Haag, R. Biomacromolecules 2021, 22, 1545-1554.
[35] Brooks, D.; Kizhakkedathu, J.; Shenoi, R.; Weinhart, M.; Lai, B.; Haag, R.; Groeger, D. 2019, US Patent- 10392474.
[36] T. Crouzier, K. Boettcher, A. R. Geonnotti, N. L. Kavanaugh, J. B. Hirsch, K. Ribbeck, O. Lieleg, Adv. Mater. Interfaces 2015, 2, 1500308.
[37] Petrou, G.; Crouzier, Biomater. Sci. 2018, 6, 2282-2297
[38] Cook, M. T.; Smith, S. L.; Khutoryanskiy, V. V.; Chem. Commun. 2015, 51, 14447-14450
[39] Marczynski, M.; Kimna, C.; Lieleg, O. Adv. Drug Deliv. Rev. 2021, 178, 113845.
[40] S. Y. Kim, W. Jin, A. Sood, D. W. Montgomery, O. C. Grant, M. M. Fuster, L. Fu, J. S. Dordick, R. J. Woods, F. Zhang, R. J. Linhardt, Antiviral Res. 2020, 181, 104873.
[41] O. Alfi, I. From, A. Yakirevitch, M. Drendel, M. Wolf, K. Meir, Z. Zakay-Rones, Y. Nevo, S. Elgavish, O. Ilan, Y. Weisblum, S. Tayeb, M. Gross, W. Jonas, J. Ives, M. Oberbaum, A. Panet, D. G. Wolf, J. Virol. 2020, 94, e01258-20
[42] G. Gunkel, M. Weinhart, T. Becherer, R. Haag, W. T. S. Huck, Biomacromolecules 2011, 12, 4169-4172
[43] P. Dey, P. Rajdev, P. Pramanik, R. Haag, S. Ghosh, Macromolecules 2020, 53, 7044-7052.

## Claims

1. Polymer having a structure according to general formula (I): 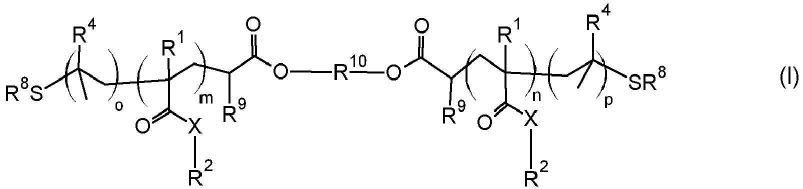 wherein
R¹ = -CH₃ or -H,
R² = 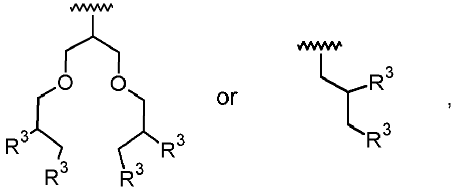
R³ = independently from other R³ residues in the same molecule: -OH, -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 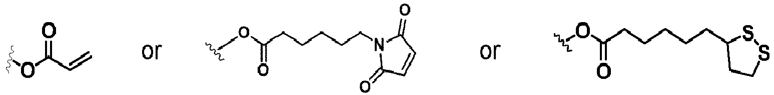 or a salt of any of these residues,
R⁴ = independently from other R⁴ residues in the same molecule: 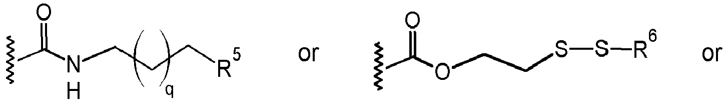 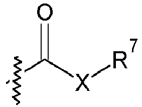
R⁵ = independently from other R⁵ residues in the same molecule: 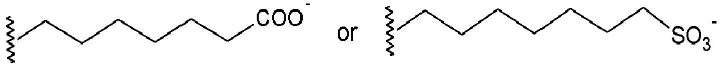 or 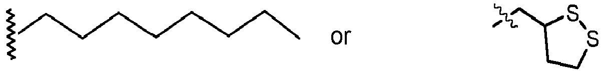 or 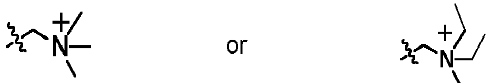 or a salt of any of these residues,
R⁶ = independently from other R⁶ residues in the same molecule: 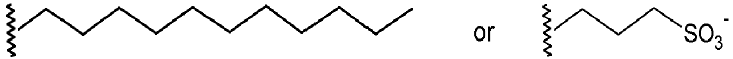 or 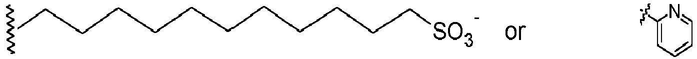 or 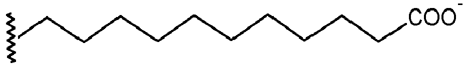 or a salt of any of these residues,
R⁷ = independently from other R⁷ residues in the same molecule: 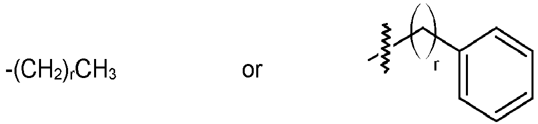 or 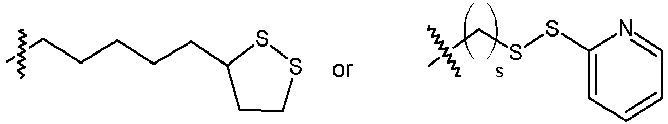 or 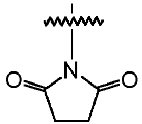
R⁸ = independently from other R⁸ residues in the same molecule: 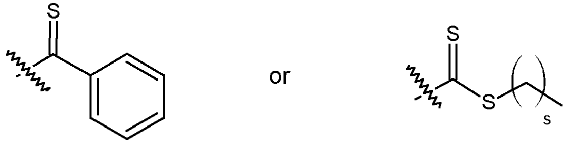 or 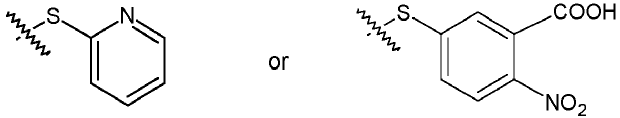 or 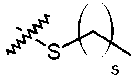
R⁹ = independently from other R⁹ residues in the same molecule: a methyl residue or a phenyl residue,
R¹⁰ = independently from other R¹⁰ residues in the same molecule: a substituted or non-substituted C₁-C₁₂ alkyl residue or a substituted or non-substituted C₆-C₁₂ aryl residue,
X = independently from other X residues in the same molecule: -O or -NH,
m = 10 to 500,
n = 10 to 500,
o = 0 to 50,
p = 0 to 50,
q = independently from other q indices in the same molecule: 1 to 10,
r = independently from other r indices in the same molecule: 0 to 11, and
s = independently from other s indices in the same molecule: 1 to 12.

2. Polymer according to claim 1, **characterized in that** a degree of functionalization of the polymer, defined by R³ residues being different from -OH, lies in a range of from 10 to 100 %.

3. Polymer according to claim 1 or 2, **characterized in that** R³ is independently from other R³ residues in the same molecule -OH, -OSO₃⁻, -O(CH₂)_{q}SO₃⁻, -(CH₂)_{q}SO₃⁻, -OPO₃²⁻, -OCO(CH₂)_{q}COO⁻ or a salt of any of these residues.

4. Polymer according to any of the preceding claims, **characterized in that** o and p are 0.

5. Polymer according to any of the preceding claims, **characterized in that** X is -O.

6. Hydrogel, obtainable by crosslinking a polymer according to any of the preceding claims by ultraviolet light or with a crosslinker chosen from the group consisting of a PEG-dithiol, a 4-arm PEG-tetrathiol, and a PEG-dicyclooctyne corresponding to the following formula:

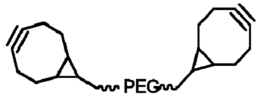

7. Use of a polymer according to any of claims 1 to 5 or a hydrogel according to claim 6 for binding viruses and/or bacteria in vitro.

8. Use according to claim 7, **characterized in that** the viruses are chosen from the group consisting of influenza viruses, coronaviruses, herpes simplex viruses, and respiratory syncytial viruses, and/or **in that** the bacteria are chosen from the group consisting of pseudomonas and Escherichia coli.

9. Use of a polymer according to any of claims 1 to 5 or a hydrogel according to claim 6 as matrix for cultivating and/or expanding cells and/or organoids in vitro.

10. Polymer according to any of claims 1 to 5 or hydrogel according to claim 6 for use in antiviral and/or antibacterial therapy of humans or animals.

11. Hydrogel according to claim 6 for use as mucus replacement, for use as cartilage replacement, or for use as synovial fluid replacement in a patient in need thereof.

12. Pharmaceutical composition comprising a polymer according to any of claims 1 to 5 or a hydrogel according to claim 6.

13. Method for manufacturing a polymer according to any of claims 1 to 5, comprising the following steps:
a) reacting a compound according to general formula (II) with a compound according to general formula (III) to obtain a compound according to general formula (IV): 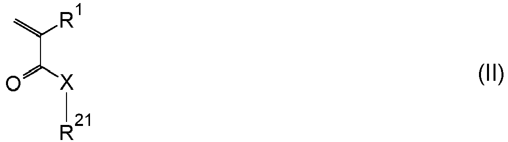 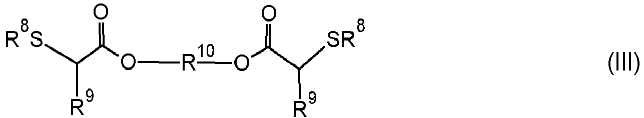 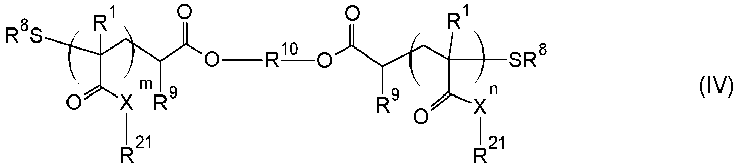 wherein
R¹ = -CH₃ or -H,
R⁸ = independently from other R⁸ residues in the same molecule: 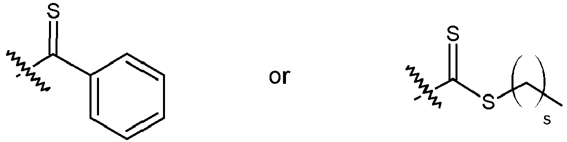 or 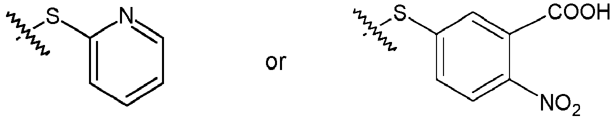 or 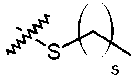
R⁹ = independently from other R⁹ residues in the same molecule: a methyl residue or a phenyl residue,
R¹⁰ = independently from other R¹⁰ residues in the same molecule: a substituted or non-substituted C₁-C₁₂ alkyl residue or a substituted or non-substituted C₆-C₁₂ aryl residue,
R²¹ = 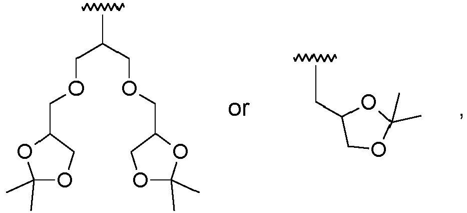
X = independently from other X residues in the same molecule: -O or -NH,
m = 10 to 500,
n = 10 to 500,
b) deprotecting the end groups of residue R²¹ to obtain a compound according to general formula (V): 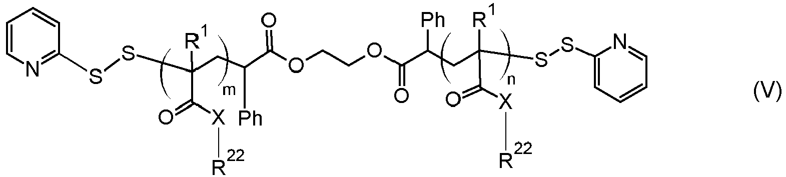 wherein
R²² = 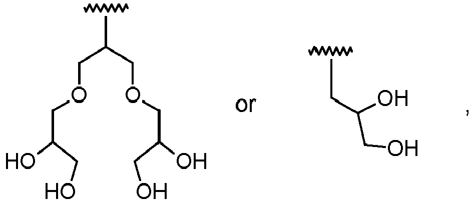
c) optionally functionalizing at least some hydroxyl groups of residue R²² with at least one of the following residues: -OSO₃⁻, -O(CH₂)_{q}SO₃⁻, -(CH₂)_{q}SO₃⁻, -OPO₃²⁻, -OCO(CH₂)_{q}COO⁻, -N₃, a sialic acid residue, a fucose residue, a galactose residue, a mannose residue, or any of the following residues: 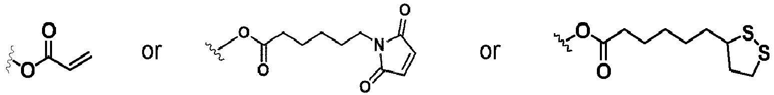 or a salt of any of these residues.

14. Method for manufacturing a polymer according to any of claims 1 to 5, comprising the following steps:
a) reacting a compound according to general formula (II) and a compound according to general formula (VII) with a compound according to general formula (III) to obtain a compound according to general formula (VIII): 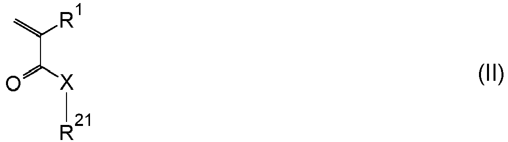 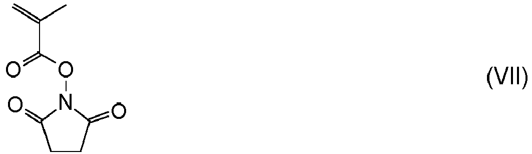 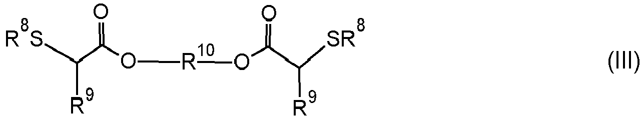 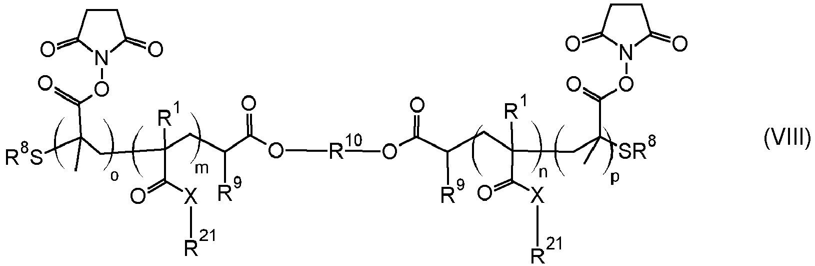 wherein
R¹ = -CH₃ or -H,
R⁸ = independently from other R⁸ residues in the same molecule: 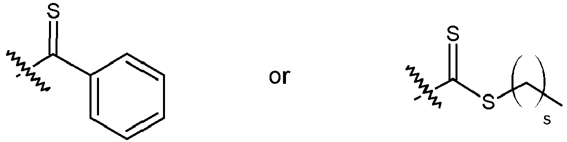 or 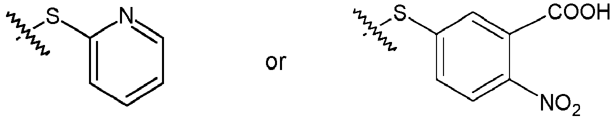 or 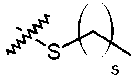
R⁹ = independently from other R⁹ residues in the same molecule: a methyl residue or a phenyl residue,
R¹⁰ = independently from other R¹⁰ residues in the same molecule: a substituted or non-substituted C₁-C₁₂ alkyl residue or a substituted or non-substituted C₆-C₁₂ aryl residue,
R²¹ = 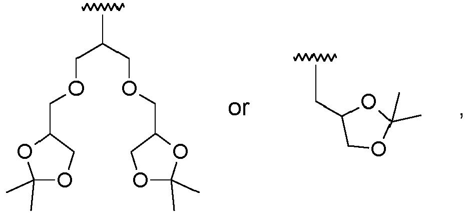
X = independently from other X residues in the same molecule: -O or -NH,
m = 10 to 500,
n = 10 to 500,
o = 0 to 50,
p = 0 to 50,
b) reacting the compound according to general formula (VIII) with a compound according to general formula (IX) to obtain a compound according to general formula (X): 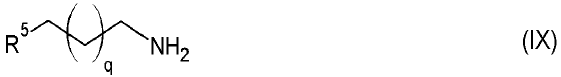 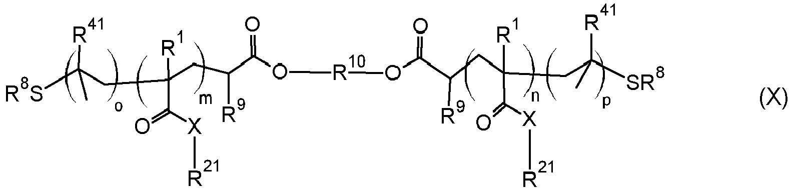 wherein
R⁴¹ = 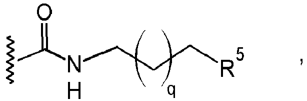
R⁵ = 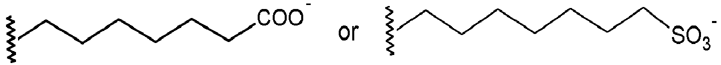 or 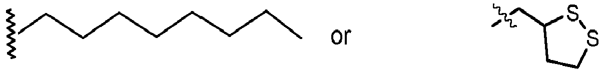 or 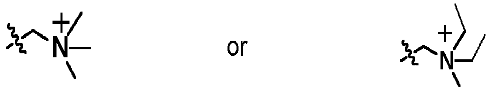 or a salt of any of these residues,
c) deprotecting the end groups of residue R²¹ to obtain a compound according to general formula (XI): 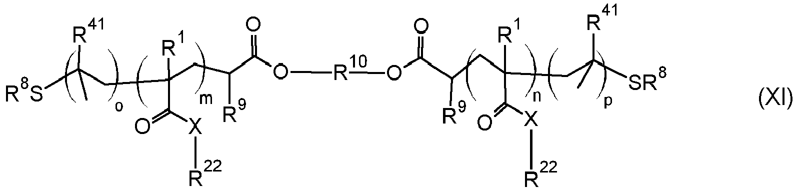 wherein
R²² = 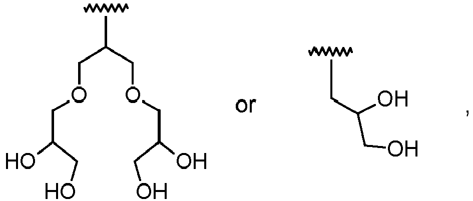
d) optionally substituting at least some hydroxyl groups of residue R²² by at least one of the following residues: -OPO₃²⁻, -O(CH₂)_{q}SO₃⁻, -(CH₂)_{q}SO₃⁻, -OCO(CH₂)_{q}COO⁻, -N₃, a sialic acid residue, a fucose residue, a galactose residue, a mannose residue, or any of the following residues: 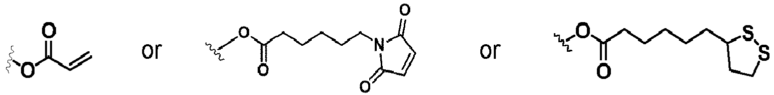 or a salt of any of these residues.

15. Method for manufacturing a polymer according to any of claims 1 to 5, comprising the following steps:
a) reacting a compound according to general formula (II) and a compound according to general formula (XII) with a compound according to general formula (III) to obtain a compound according to general formula (XIII): 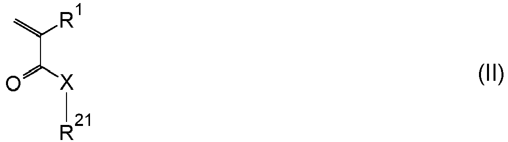 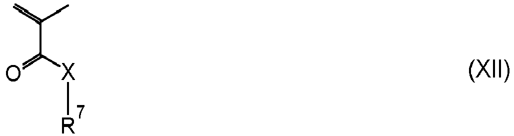 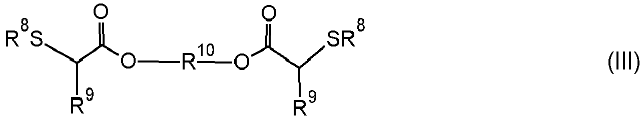 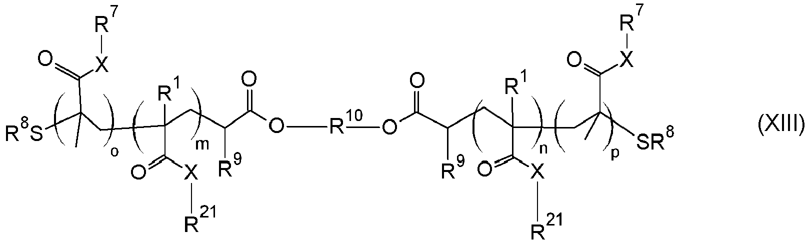 wherein
R¹ = -CH₃ or -H,
R⁷ = independently from other R⁷ residues in the same molecule: 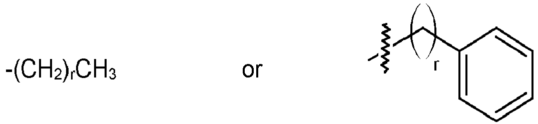 or 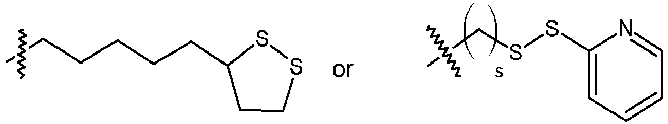 or 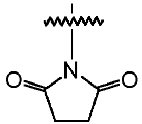
R⁸ = independently from other R⁸ residues in the same molecule: 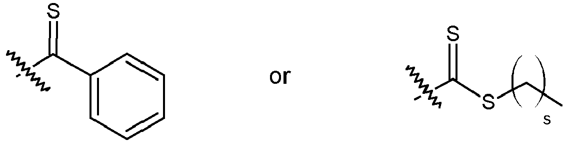 or 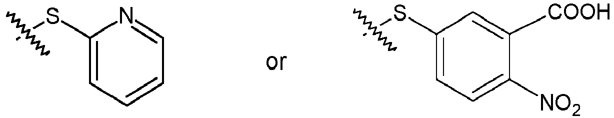 or 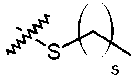
R⁹ = independently from other R⁹ residues in the same molecule: a methyl residue or a phenyl residue,
R¹⁰ = independently from other R¹⁰ residues in the same molecule: a substituted or non-substituted C₁-C₁₂ alkyl residue or a substituted or non-substituted C₆-C₁₂ aryl residue,
R²¹ = 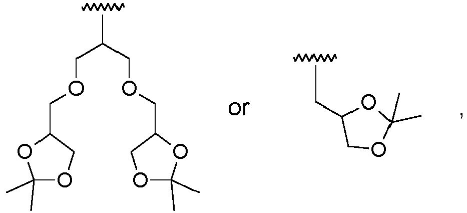
X = independently from other X residues in the same molecule: -O or -NH,
m = 10 to 500,
n = 10 to 500,
o = 0 to 50,
p = 0 to 50,
r = independently from other r indices in the same molecule: 0 to 11, and
s = independently from other s indices in the same molecule: 1 to 12.
b) deprotecting the end groups of residue R²¹ to obtain a compound according to general formula (XIV): 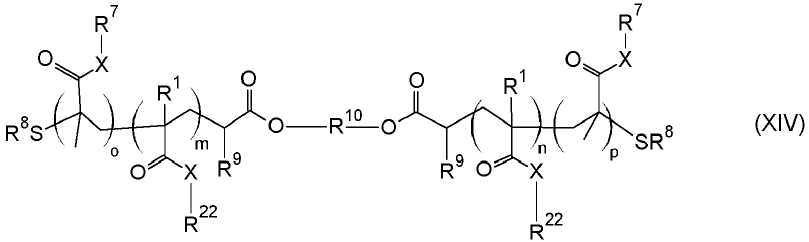 wherein
R²² = 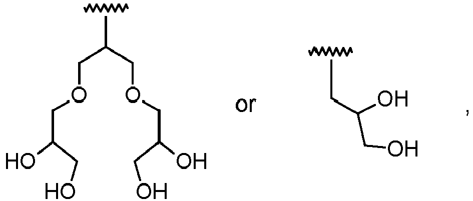
c) optionally functionalizing at least some hydroxyl groups of residue R²² with at least one of the following residues: -OPO₃²⁻, -O(CH₂)_{q}SO₃⁻, -(CH₂)_{q}SO₃⁻, -OCO(CH₂)_{q}COO⁻, -N₃, a sialic acid residue, a fucose residue, a galactose residue, a mannose residue, or any of the following residues: 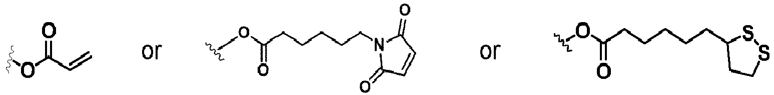 or a salt of any of these residues.
